# EUROPEAN PATENT SPECIFICATION

(11) **EP 1 161 438 B1**
(45) Date of publication and mention of the grant of the patent: **06.05.2004**
(21) Application number: 00913805.8
(22) Date of filing: 08.03.2000
(51) Int. Cl.: C07H 17/08, A61K 31/70

(54) **6-O-SUBSTITUTED MACROLIDES HAVING ANTIBACTERIAL ACTIVITY**
6-O-SUBSTITUTIERTE MAKROLIDEN MIT ANTIBAKTERIELLER WIRKUNG
MACROLIDES SUBSTITUES EN 6-O AYANT UNE ACTIVITE ANTIBACTERIENNE

(30) Priority: 15.03.1999 US 270497
(43) Date of publication of application: 12.12.2001
(73) Proprietor: ABBOTT LABORATORIES, Abbott Park, IL 60064-6050 (US)
(72) Inventor: OR, Yat, Sun, Cambridge, MA 02140 (US); CLARK, Richard, F., Mundelein, IL 60060 (US); MA, Zhenkun, Gurnee, IL 60031 (US); RUPP, Michael, J., Lake Bluff, IL 60044 (US)
(74) Representative: Modiano, Guido, Dr.-Ing.
(86) International application number: PCT/US2000/006033
(87) International publication number: WO 2000/055168

(56) References cited:
- WO-A-98/09978
- US-A- 5 866 549

## Description

### Technical Field

This invention relates to novel macrolide compounds having antibacterial activity, to pharmaceutical compositions comprising these compounds, to methods of treating bacterial infections using said compounds, and to methods of making these novel compounds.

### Background of the Invention

Erythromycins A through D are represented by the following formula:

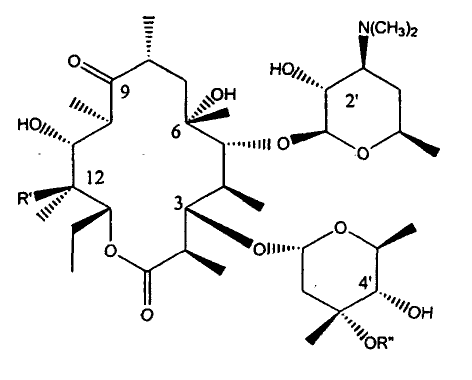

| Erythromycin | R' | R'' |
|---|---|---|
| A | -OH | -CH₃ |
| B | -H | -CH₃ |
| C | -OH | -H |
| D | -H | -H |

and are well-known and potent antibacterial agents. These compounds are used widely to treat and prevent bacterial infection. As with other antibacterial agents, however, bacterial strains having resistance or insufficient susceptibility to erythromycin have been identified. Erythromycin A has only weak activity against Gram-negative bacteria. Therefore, there is a continuing need to identify new macrolide compounds which possess improved antibacterial activity, which have less potential for developing resistance, which possess the desired Gram-negative activity, and/ or which possess unexpected selectivity against target microorganisms. Consequently, numerous investigators have prepared chemical derivatives of erythromycin in an attempt to obtain analogs having modified or improved profiles of antibiotic activity.

United States Pat. No. 5,444,051 discloses 6-O-substituted-3-oxoerythromycin A derivatives in which the. substituents are selected from alkyl, -CONH₂, - CONHC(O)alkyl and -CONHSO₂alkyl. WO 97/10251, published March 20, 1997, discloses 6-0-methyl 3-descladinose erythromycin derivatives. European Patent Application 596802, published May 11, 1994, discloses bicyclic 6-O-methyl-3-oxoerythromycin A derivatives. WO 92/09614, published June 11, 1992, discloses tricyclic 6-O-methylerythromycin A derivatives. WO 98/09978 and United States Patent No. 5,866,549 disclose 6-O-substituted ketolides having antibacterial activity.

### Summary of the Invention

The present invention relates to compounds selected from the group consisting of
a compound of formula I 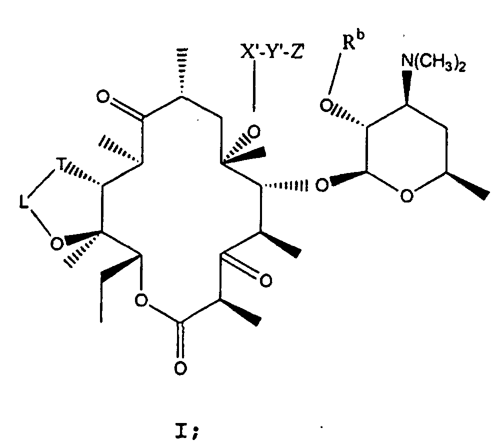 a compound of formula II 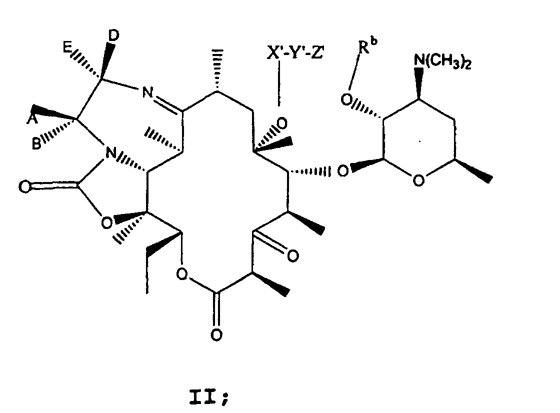 and a compound of formula III 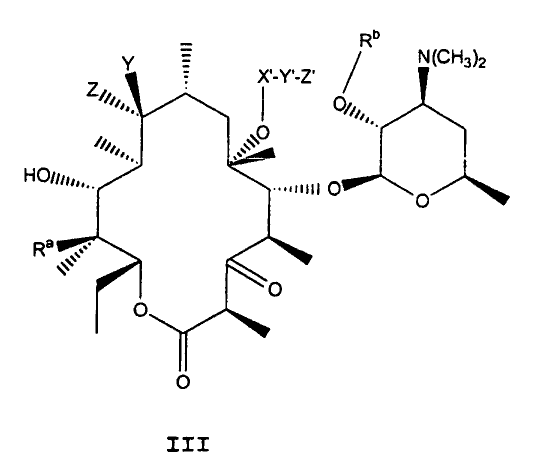 or a pharmaceutically acceptable salt, solvate, ester or prodrug thereof, wherein either:
(a) Y and Z taken together define a group X, and X is selected from the group consisting of
   (1) =O,
   (2) =N-OH,
   (3) =N-O-R¹ where R¹ is selected from the group consisting of
      (a) unsubstituted C₁-C₁₂ alkyl,
      (b) C₁-C₁₂ alkyl substituted with aryl,
      (c) C₁-C₁₂ alkyl substituted with substituted aryl,
      (d) C₁-C₁₂ alkyl substituted with heteroaryl,
      (e) C₁-C₁₂ alkyl substituted with substituted heteroaryl,
      (f) C₃-C₁₂ cycloalkyl, and
      (g) -Si-(R²)(R³)(R⁴) wherein R², R³ and R⁴ are each independently selected from C₁-C₁₂ alkyl and aryl;
         and
   (4) =N-O-C(R⁵) (R⁶)-O-R¹ where R¹ is as previously defined and R⁵ and R⁶ are each independently selected from the group consisting of
      (a) hydrogen,
      (b) unsubstituted C₁-C₁₂ alkyl,
      (c) C₁-C₁₂ alkyl substituted with aryl,
      (d) C₁-C₁₂ alkyl substituted with substituted aryl,
      (e) C₁-C₁₂ alkyl substituted with heteroaryl,
         and
      (f) C₁-C₁₂ alkyl substituted with substituted heteroaryl,
      or R⁵ and R⁶ taken together with the atom to which they are attached form a C₃-C₁₂ cycloalkyl ring;
   or
(b) one of Y and Z is hydrogen and the other is selected from the group consisting of
   (1) hydrogen,
   (2) hydroxy,
   (3) protected hydroxy,
      and
   (4) NR⁷R⁸ wherein R⁷ and R⁸ are independently selected from hydrogen and C₁-C₆ alkyl, or R⁷ and R⁸ are taken with the nitrogen atom to which they are connected to form a 3- to 7-membered ring which, when the ring is a 5- to 7-membered ring, may optionally contain a hetero function selected from the group consisting of -O-, -NH-, - N(C₁-C₆-alkyl-)-, -N(aryl)-, -N(aryl-C₁-C₆-alkyl-)-, - N(substituted-aryl-C₁-C₆-alkyl-)-, -N(heteroaryl)-, - N(heteroaryl-C₁-C₆-alkyl-)-, -N(substituted-heteroaryl-C₁-C₆-alkyl-)-, and -S- or -S(O)ₙ-, wherein n is 1 or 2;
   R^{a} is hydrogen or hydroxy;
   R^{b} is hydrogen or a hydroxy protecting group;
   L is methylene or carbonyl, provided that when L is methylene, T is -O-;
   T is selected from the group consisting of -O-, -NH-, and -N(W-R^{d})-, wherein W is absent or is selected from the group consisting of -O-, -NH-CO-, -N=CH- and -NH-, and R^{d} is selected from the group consisting of
   (1) hydrogen,
   (2) C₁-C₆-alkyl optionally substituted with one or more substituents selected from the group consisting of
      (a) aryl,
      (b) substituted aryl,
      (c) heteroaryl,
      (d) substituted heteroaryl,
      (e) hydroxy,
      (f) C₁-C₆-alkoxy,
      (g) NR⁷R⁸, wherein R⁷ and R⁸ are as defined previously,
         and
      (h) -CH₂-M-R⁹,
         wherein M is selected from the group consisting of:
         (i) -C(O)-NH-,
         (ii) -NH-C(O)-,
         (iii) -NH-,
         (iv) -N=,
         (v) -N(CH₃)-,
         (vi) -NH-C(O)-O-
         (vii) -NH-C(O)-NH-
         (viii) -O-C(O)-NH-
         (ix) -O-C(O)-O-
         (x) -O-,
         (xi) -S(O)ₙ-, wherein n is 0, 1 or 2,
         (xii) -C(O)-O-,
         (xiii) -O-C(O)-,
            and
         (xiv) -C(O)-;
            and
         wherein R⁹ is selected from the group consisting of:
      (i) C₁-C₆ alkyl, optionally substituted with a substituent selected from the group consisting of
         (aa) aryl,
         (bb) substituted aryl,
         (cc) heteroaryl, and
         (dd) substituted heteroaryl;
      (ii) aryl,
      (iii) substituted aryl,
      (iv) heteroaryl,
      (v) substituted heteroaryl,
         and
      (vi) heterocycloalkyl;
   (3) C₃-C₇ cycloalkyl,
   (4) aryl,
   (5) substituted aryl,
   (6) heteroaryl,
      and
   (7) substituted heteroaryl;
   X' is
   C₃ alkynyl;
   Y' is thienyl;
   Z' is pyridyl;
   and A, B, D and E are independently selected from the group consisting of:
   (a) hydrogen;
   (b) C₁-C₆ alkyl, optionally substituted with one or more substituents selected from the group consisting of:
      (i) aryl;
      (ii) substituted aryl;
      (iii) heteroaryl;
      (iv) substituted heteroaryl;
      (v) heterocycloalkyl;
      (vi) hydroxy;
      (vii) C₁-C₆ alkoxy;
      (viii) halogen consisting of Br, Cl, F or I; and
      (ix) NR⁷R⁸, wherein R⁷ and R⁸ are as previously defined;
   (c) C₃-C₇ cycloalkyl;
   (d) aryl;
   (e) substituted aryl;
   (f) heteroaryl;
   (g) substituted heteroaryl;
   (h) heterocycloalkyl; and
   (i) a group selected from option (b) above further substituted with -M-R⁹, wherein M and R⁹ are as previously defined, with the proviso that at least two of A, B, D and E are hydrogen,;
   or any one pair of substituents, consisting of AB, AD, AE, BD, BE or DE, is taken together with the atom or atoms to which they are attached to form a 3- to 7-membered ring optionally containing a hetero function selected from the group consisting of -O-, -NH-, -N(C₁-C₆ alkyl-)-, -N(aryl-C₁-C₆ alkyl-)-, -N(substituted aryl-C₁-C₆ alkyl-)-, -N(heteroaryl-C₁-C₆ alkyl-)-, -N(substituted heteroaryl- C₁-C₆ alkyl-)-, -S- or -S(O)ₙ-, wherein n is 1 or 2, -C(O)-NH, -C(O)-NR¹²-, wherein R¹² is selected from the group consisting of hydrogen, C₁-C₃ alkyl, C₁-C₃ alkyl substituted with aryl, substituted aryl, heteroaryl, or substituted heteroaryl, -NH-C(O)-, and -NR¹²-C(O)-.

The present invention also relates to pharmaceutical compositions containing a pharmaceutically effective amount of a compound of formulae I, II or III as defined above in combination with a pharmaceutically acceptable carrier.

The present invention further relates to a process for preparing compounds of formula I, II, or III,
wherein said process comprises:
coupling a compound selected from the group consisting of a compound of formula I^{a} 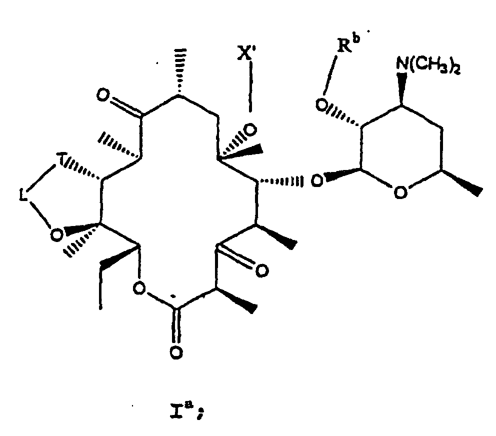
a compound of formula II^{a} 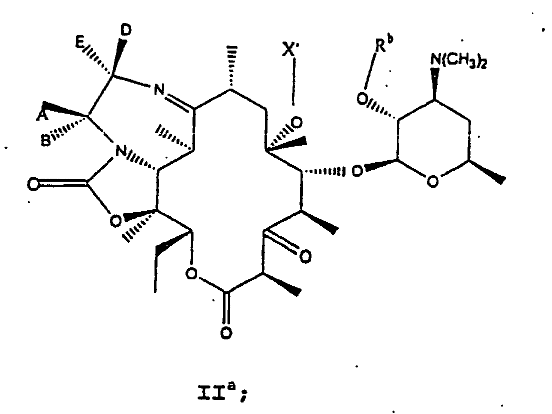
and a compound of formula III^{a} 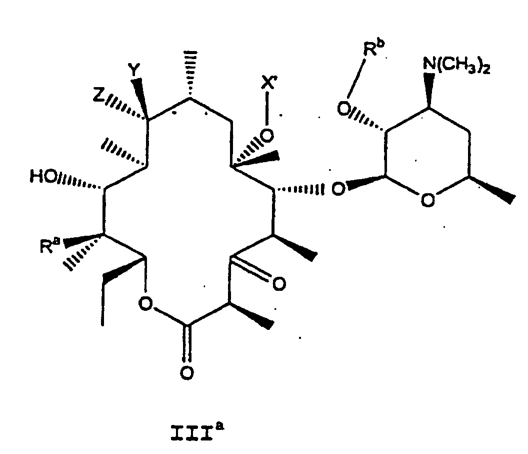
wherein
R^{b} is a hydroxy protecting group, and
Y, Z, X, R¹, R², R³, R⁴, R⁵, R⁶, R⁷, R⁸, n, R^{a}, L, T, R^{d}, W, M, R⁹, X', Y', Z', A, B, D, E, R¹² are as previously defined;
with a compound selected from the group consisting of X¹-Y'-Z' and X¹-Y'-X², wherein X¹ and X² are independently selected from the group consisting of bromide, iodide, sulfonate, trialkylstannane, boronic acid, and borate, and Y' and Z' are as previously defined;
in the presence of a palladium catalyst, with the proviso that when X¹-Y'-X² is employed, a subsequent coupling reaction is performed with X³-Z', wherein X³ is selected from the group consisting of bromide, iodide, sulfonate, trialkylstannane, boronic acid, and borate, and Z' is as previously defined;
(b) optionally deprotecting;
and
(c) optionally isolating the desired compound.

The invention still further relates to the use of a therapeutically effective amount of a compound of formula I, II or III as defined above for treating bacterial infections in a host mammal in need of such treatment.

### Detailed Description of the Invention

As used throughout the specification and the claims, the following terms have the meanings specified.

The terms "C₁-C₃ alkyl", "C₁-C₆ alkyl", and "C₁-C₁₂ alkyl" as used herein refer to saturated, straight, or branched chain hydrocarbon radicals derived from a hydrocarbon moiety containing between one and three, one and six, and one and twelve carbon atoms, respectively, by removal of a single hydrogen atom. Examples of C₁-C₃ alkyl radicals include methyl, ethyl, propyl, and isopropyl, examples of C₁-C₆ alkyl radicals include methyl, ethyl, propyl, isopropyl, n-butyl, tert-butyl, neopentyl, and n-hexyl. Examples of C₁-C₁₂ alkyl radicals include all the foregoing examples, as well as n-heptyl, n-octyl, n-nonyl, n-decyl, n-undecyl, and n-docecyl.

The term "C₁-C₆ alkoxy" as used herein refers to an C₁-C₆ alkyl group, as previously defined, attached to the parent molecular moiety through an oxygen atom. Examples of C₁-C₆ alkoxy include methoxy, ethoxy, propoxy, isopropoxy, n-butoxy, tert-butoxy, neopentoxy and n-hexoxy.

The term "C₃-C₁₂ alkenyl" denotes a monovalent or divalent group derived from a hydrocarbon moiety containing from two to twelve carbon atoms and having at least one carbon-carbon double bond by the removal of a single hydrogen atom. Alkenyl groups include propenyl, butenyl, 1-methyl-2-buten-1-yl, and the like.

The term "C₃-C₁₂ alkynyl" as used herein refers to a monovalent or divalent group derived from a hydrocarbon containing from two to twelve carbon atoms and having at least one carbon-carbon triple bond by the removal of a single hydrogen atom. Representative alkynyl groups include 2-propynyl (propargyl), 1-propynyl, and the like.

The term "alkylene" denotes a divalent group derived from a straight or branched chain saturated hydrocarbon by the removal of two hydrogen atoms, for example methylene, 1,2-ethylene, 1,1-ethylene, 1,3-propylene, 2,2-dimethylpropylene, and the like.

The term "C1-C3-alkylamino" as used herein refers to one or two C1-C3-alkyl groups, as previously defined, attached to the parent molecular moiety through a nitrogen atom. Examples of C1-C3-alkylamino include methylamino, dimethylamino, ethylamino, diethylamino, and propylamino.

The term "oxo" denotes a group wherein two hydrogen atoms on a single carbon atom in an alkyl group as defined above are replaced with a single oxygen atom (for example, a carbonyl group).

The term "aprotic solvent" as used herein refers to a solvent that is relatively inert to proton activity, for example, not acting as a proton-donor. Examples include hydrocarbons, such as hexane and toluene, for example, halogenated hydrocarbons, such as, for example, methylene chloride, ethylene chloride, chloroform, and the like, heteroaryl compounds, such as, for example, tetrahydrofuran and N-methylpyrrolidinone, and ethers such as diethyl ether, bis-methoxymethyl ether. Such compounds are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of aprotic solvents may be found in organic chemistry textbooks or in specialized monographs, for example: Organic Solvents Physical Properties and Methods of Purification, 4th ed., edited by John A. Riddick et al., Vol. II, in the Techniques of Chemistry Series, John Wiley & Sons, NY, 1986.

The term "aryl" as used herein refers to a mono- or bicyclic carbocyclic ring system having one or two aromatic rings including phenyl, naphthyl, tetrahydronaphthyl, indanyl, indenyl, and the like. Aryl groups (including bicyclic aryl groups) can be unsubstituted or substituted with one, two or three substituents independently selected from loweralkyl, substituted loweralkyl, haloalkyl, alkoxy, thioalkoxy, amino, alkylamino, dialkylamino, acylamino, cyano, hydroxy, halo, mercapto, nitro, carboxaldehyde, carboxy, alkoxycarbonyl and carboxamide. In addition, substituted aryl groups include tetrafluorophenyl and pentafluorophenyl.

The term "C3-C12-cycloalkyl" denotes a monovalent group derived from a monocyclic or bicyclic saturated carbocyclic ring compound by the removal of a single hydrogen atom. Examples include cyclopropyl, cyclobutyl, cyclopentyl, cyclohexyl, bicyclo[2.2.1]heptyl, and bicyclo[2.2.2]octyl.

The terms "halo" and "halogen" as used herein refer to an atom selected from fluorine, chlorine, bromine and iodine.

The term "alkylamino" refers to a group having the structure -NHR', wherein R' is alkyl, as previously defined. Examples of alkylamino include methylamino, ethylamino, iso-propylamino, and the like.

The term "dialkylamino" refers to a group having the structure -NR'R" wherein R' and R" are independently selected from alkyl, as previously defined. Additionally, R' and R" taken together may optionally be - (CH2)k- where k is an integer of from 2 to 6. Examples of dialkylamino include, dimethylamino, diethylaminocarbonyl, methylethylamino, piperidino, and the like.

The term "haloalkyl" denotes an alkyl group, as defined above, having one, two, or three halogen atoms attached thereto and is exemplified by such groups as chloromethyl, bromoethyl, trifluoromethyl, and the like.

The term "alkoxycarbonyl" represents an ester group, for example, an alkoxy group, attached to the parent molecular moiety through a carbonyl group such as methoxycarbonyl, ethoxycarbonyl, and the like.

The term "thioalkoxy" refers to an alkyl group as previously defined attached to the parent molecular moiety through a sulfur atom.

The term "carboxaldehyde" as used herein refers to a group of formula -CHO.

The term "carboxy" as used herein refers to a group of formula -CO2H.

The term "carboxamide" as used herein refers to a group of formula -CONHR'R" wherein R' and R" are independently selected from hydrogen or alkyl, or R' and R" taken together may optionally be -(CH2)k-, where k is an integer from 2 to 6.

The term "heteroaryl", as used herein, refers to a cyclic aromatic radical having from five to ten ring atoms of which one ring atom is selected from S, O and N; one, two, or three ring atoms may be additional heteroatoms independently selected from S, O and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isooxazolyl, thiadiazolyl, oxadiazolyl, tetrazolyl, thiophenyl, furanyl, quinolinyl, isoquinolinyl, and the like.

The term "heterocycloalkyl" as used herein, refers to a non-aromatic partially unsaturated or fully saturated 3- to 10-membered ring system, which includes single rings of 3 to 8 atoms in size and bi- or tricyclic ring systems which may include aromatic six-membered aryl or heteroaryl rings fused to a non-aromatic ring. These heterocycloalkyl rings include those having from one to three heteroatoms independently selected from oxygen, sulfur and nitrogen, in which the nitrogen and sulfur heteroatoms may optionally be oxidized and the nitrogen heteroatom may optionally be quaternized.

Representative heterocycles include pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl.

Specific heterocycloalkyl rings include 3-methylpiperidine, 4-(diphenylmethyl)piperazine, 4-(2-(bis-(2-propenyl)amino)ethyl)piperazine, 4-(2-(diethylamino)ethyl)piperazine, 4-(2-chlorophenyl)piperazine, 4-(3,4-dimethoxyphenyl)piperazine, 4-(4-nitrophenyl)piperazine, 4-(4-trifluoromethylphenyl)piperazine, 4-hydroxy-4-phenylpiperidine, 4-hydroxypyrrolidine, 4-methylpiperazine, 4-phenylpiperazine, 4-piperidinylpiperazine, 4-((2-furanyl)carbonyl)piperazine, 4-((1,3-dioxolan-5-yl)methyl)piperazine, 6-fluoro-1,2,3,4-tetrahydro-2-methylquinoline, 1,4-diazacycloheptane, 2,3-dihydroindolyl, 3,3-dimethylpiperidine, 1,2,3,4-tetrahydroisoquinoline, 1,2,3,4-tetrahydroquinoline, azacyclooctane, decahydroquinoline, piperazine, piperidine, pyrrolidine, thiomorpholine, triazole, and the like.

The term "heteroarylalkyl" as used herein, refers to a heteroaryl group as defined above attached to the parent molecular moiety through an alkylene group wherein the alkylene group is of one to four carbon atoms.

"Hydroxy-protecting group", as used herein, refers to an easily removable group which is known in the art to protect a hydroxyl group against undesirable reaction during synthetic procedures and to be selectively removable. The use of hydroxy-protecting groups is well known in the art for protecting groups against undesirable reactions during a synthetic procedure and many such protecting groups are known, cf., for example, Greene and Wuts, Protective Groups in Organic Synthesis, 2nd edition, John Wiley & Sons, New York (1991). Examples of hydroxy-protecting groups include methylthiomethyl, tert-dimethylsilyl, tert-butyldiphenylsilyl, ethers such as methoxymethyl, and esters including acetyl benzoyl, and the like.

The term "ketone protecting group", as used herein, refers to an easily removable group which is known in the art to protect a ketone group against undesirable reactions during synthetic procedures and to be selectively removable. The use of ketone-protecting groups is well known in the art for protecting groups against undesirable reactions during a synthetic procedure and many such protecting groups are known. *See*, for examples, Greene and Wuts, Protective Groups in Organic Synthesis, 2nd edition, John Wiley & Sons, New York (1991). Examples of ketone-protecting groups include ketals, oximes, O-substituted oximes for example 0-benzyl oxime, O-phenylthiomethyl oxime, 1-isopropoxycyclohexyl oxime, and the like.

The term "protected-hydroxy" refers to a hydroxy group protected with a hydroxy protecting group, as defined above, including benzoyl, acetyl, trimethylsilyl, triethylsilyl, methoxymethyl groups, for example.

The term "substituted aryl" as used herein refers to an aryl group as defined herein substituted by independent replacement of one, two or three of the hydrogen atoms thereon with Cl, Br, F, I, OH, CN, C₁-C₃ alkyl, C₁-C₆ alkoxy, C₁-C₆ alkoxy substituted with aryl, haloalkyl, thioalkoxy, amino, alkylamino, dialkylamino, mercapto, nitro, carboxaldehyde, carboxy, alkoxycarbonyl and carboxamide. In addition, any one substitutent may be an aryl, heteroaryl, or heterocycloalkyl group. Substituted aryl groups also include tetrafluorophenyl and pentafluorophenyl.

The term "substituted heteroaryl" as used herein refers to a heteroaryl group as defined herein substituted by independent replacement of one, two or three of the hydrogen atoms thereon with Cl, Br, F, I, OH, CN, C₁-C₃ alkyl, C₁-C₆ alkoxy, C₁-C₆ alkoxy substituted with aryl, haloalkyl, thioalkoxy, amino, alkylamino, dialkylamino, mercapto, nitro, carboxaldehyde, carboxy, alkoxycarbonyl and carboxamide. In addition, any one substitutent may be an aryl, heteroaryl, or heterocycloalkyl group.

The term "substituted heterocycloalkyl" as used herein, refers to a heterocycloalkyl group, as defined above, substituted by independent replacement of one, two or three of the hydrogen atoms thereon with Cl, Br, F, I, OH, CN, C₁-C₃ alkyl, C₁-C₆ alkoxy, C₁-C₆ alkoxy substituted with aryl, haloalkyl, thioalkoxy, amino, alkylamino, dialkylamino, mercapto, nitro, carboxaldehyde, carboxy, alkoxycarbonyl and carboxamide. In addition, any one substitutent may be an aryl, heteroaryl, or heterocycloalkyl group.

Numerous asymmetric centers may exist in the compounds of the present invention. Except where otherwise noted, the present invention contemplates the various stereoisomers and mixtures thereof. Accordingly, whenever a bond is represented by a wavy line, it is intended that a mixture of stereoorientations or an individual isomer of assigned or unassigned orientation may be present.

Preferred for the practice of the instant invention are those compounds of formulae I, II, and III wherein X', Y', and Z' combine to form a group R, and R is selected from the group consisting of
-(CH₂)-C≡C-(5-(2-pyridyl)-2-thienyl),
-(CH₂)-C≡C-(5-(3-pyridyl)-2-thienyl), and
-(CH₂)-C≡C-(5-(4-pyridyl)-2-thienyl).
-C(H)=CH-(2-(5-bromo-(1,3-thiazol-2-yl))-5-(1,3-thiazolyl)),
-C(H)=CH-(2-(5-bromo-1,3-thiazol-2-yl)-5-(1,3-thiazolyl)),
-C(H)=CH-(2-(2-thienyl)-5-thiazolyl),
-C(H)=CH-(2-(2-pyrazinyl)-5-(1,3-thiazolyl)),
-C(H)=CH-(2-(5-pyrimidinyl)-5-(1,3-thiazolyl)),
-C(H)=CH-(2-(5-(1,3-thiazol-5-yl)-5-(1,3-thiazolyl)),
-C(H)=CH-(5-(2-pyrimidinyl)-2-thienyl),
-C(H)=CH-(5-(2-pyrazinyl)-2-thienyl),
-C(H)=CH-(5-(2-(1,3-thiazolyl)-2-thienyl),
-C(H)=CH-(5-(4-pyrimidinyl)-2-thienyl),
-C(H)=CH-(4-(3-pyridyl)-2-(1,3-thiazolyl)),
-C(H)=CH-(4-(3-pyridyl)-2-(1,3-thiazolyl)),
-C(H)=CH-(4-(3-pyridyl)-2-(1,3-thiazolyl)),
-C(H)=CH-(4-(2-thienyl)-2-(1,3-thiazolyl)),
-C(H)=CH-(5-(3-pyridyl)-2-thienyl),
-C(H)=CH-(5-(2-pyrazinyl)-2-thienyl),
-C(H)=CH-(5-(5-pyrimidinyl)-2-thienyl),
-C(H)=CH-(5-(3,4-dichlorophenyl)-2-thienyl),
-C(H)=CH-(5-(3-fluorophenyl)-2-thienyl),
-C(H)=CH-(5-(5-(1,3-thiazoyl))-2-thienyl),
-C(H)=CH-(2,2'-bisthienyl),
-C(H)=CH-(5-(2-pyridyl)-2-thienyl),
-C(H)=CH-(5-(3-thienyl)-2-thienyl), and
-C(H)=CH-(5-(2-furanyl)-2-thienyl).

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al*. describe pharmaceutically acceptable salts in detail in *J. Pharmaceutical Sciences,* 66: 1-19 (1977). The salts can be prepared *in situ* during the final isolation and purification of the compounds of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As used herein, the term "pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyrates, acrylates and ethylsuccinates.

The term "pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed in vivo to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

Representative compounds of the present invention were assayed *in vitro* for antibacterial activity as follows: Twelve petri dishes containing successive aqueous dilutions of the test compound mixed with 10 mL of sterilized Brain Heart Infusion (BHI) agar (Difco 0418-01-5) were prepared. Each plate was inoculated with 1:100 (or 1:10 for slow-growing strains, such as Micrococcus and Streptococcus) dilutions of up to 32 different microorganisms, using a Steers replicator block. The inoculated plates were incubated at 35-37 °C for 20 to 24 hours. In addition, a control plate, using BHI agar containing no test compound, was prepared and incubated at the beginning and end of each test.

An additional plate containing a compound having known susceptibility patterns for the organisms being tested and belonging to the same antibiotic class as the test compound was also prepared and incubated as a further control, as well as to provide test-to-test comparability. Erythromycin A was used for this purpose.

After incubation, each plate was visually inspected. The minimum inhibitory concentration (MIC) was defined as the lowest concentration of drug yielding no growth, a slight haze, or sparsely isolated colonies on the inoculum spot as compared to the growth control. The results of this assay, shown below in Table 1, demonstrate the antibacterial activity of the compounds of the invention.

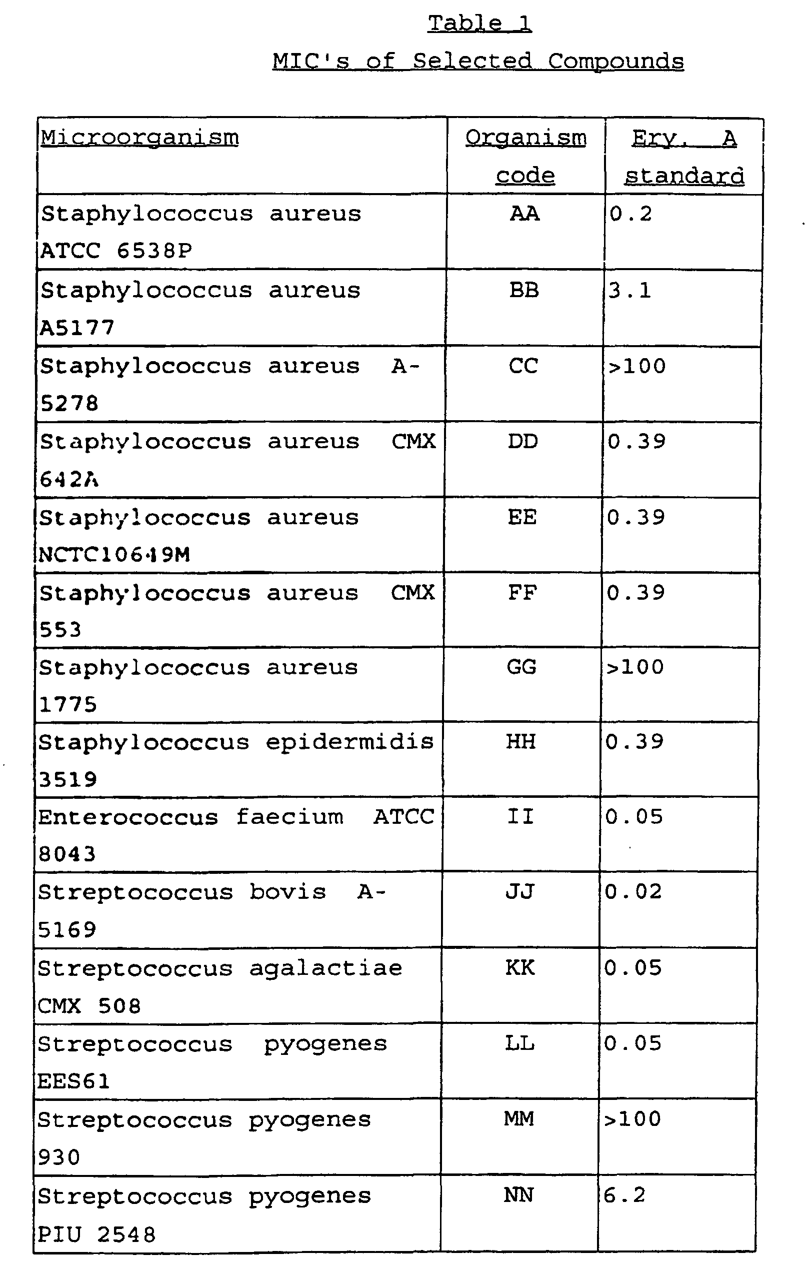

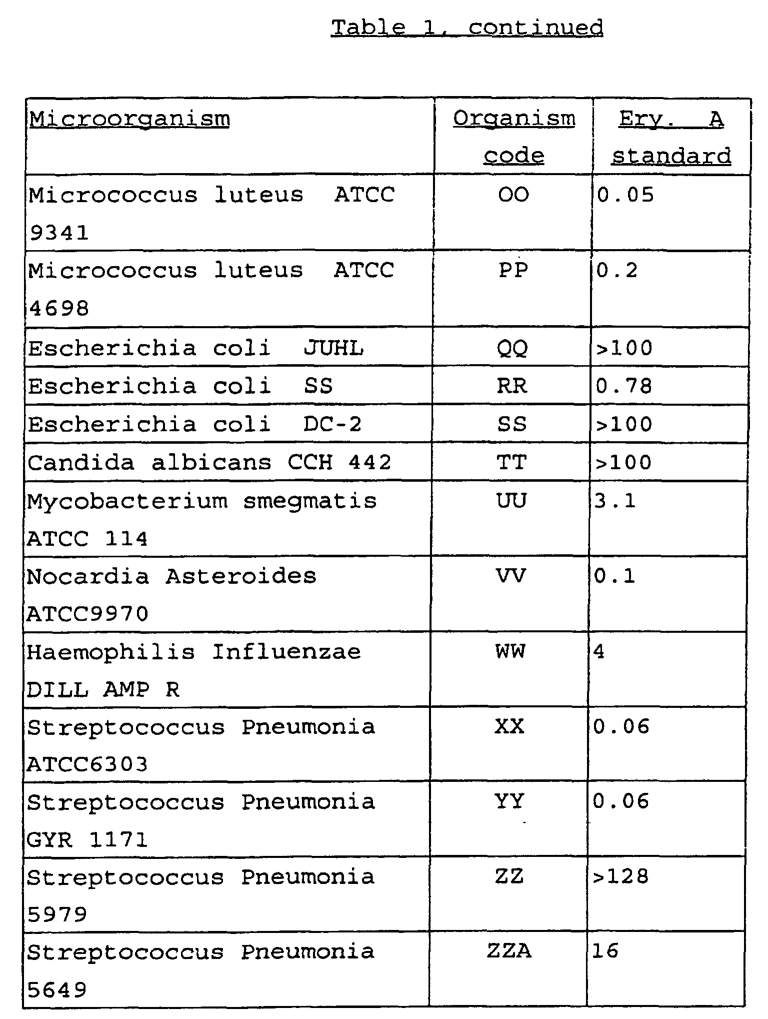

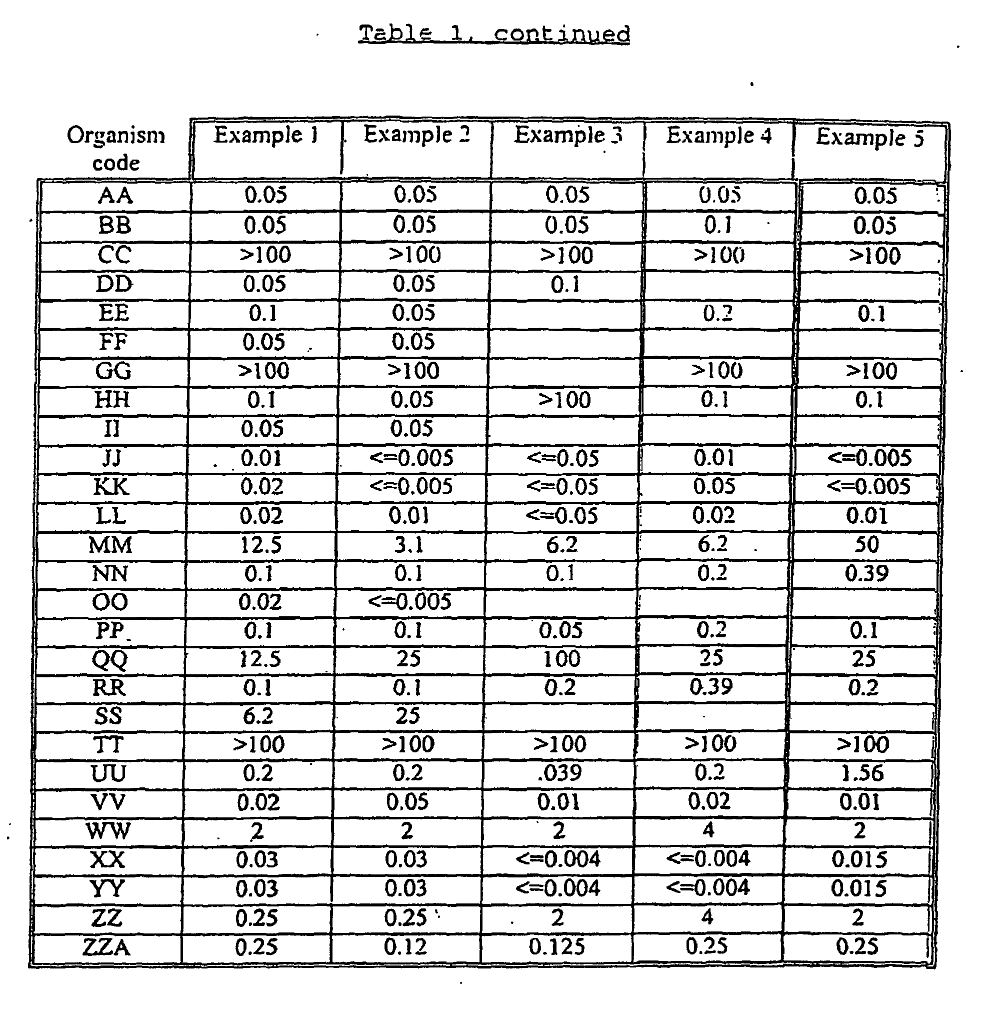

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactidepolyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable nonirritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polethylene glycols and the like.

The active compounds can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eyd ns are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Bacterial infections are treated or prevented in a patient such as a human or lower mammal by administering to the patient a therapeutically effective amount of a compound of the invention, in such amounts and for such time as is necessary to achieve the desired result. By a "therapeutically effective amount" of a compound of the invention is meant a sufficient amount of the compound to treat bacterial infections, at a reasonable benefit/risk ratio applicable to any medical treatment. It will be understood, however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens according to the present invention comprise administration to a patient in need of such treatment from about 10 mg to about 2000 mg of the compound(s) of this invention per day in single or multiple doses.

Abbreviations used in the Schemes and Examples include the following: Ac for acetate; dba for dibenzylidine acetone; THF for tetrahydrofuran; DME for dimethoxy ethane; DMF for N,N-dimethylformamide; TFA for trifluoroacetic acid; DMSO for dimethylsulfoxide; and TMS for trimethylsilyl. Starting materials, reagents and solvents were purchased from Aldrich Chemical.Company (Milwaukee, WI).

The compounds and processes of the present invention will be better understood in connection with the following synthetic Schemes, which illustrate the methods by which the compounds of the invention may be prepared. The compounds of formulae I, II, and III may be prepared by a variety of synthetic routes. Representative procedures are shown below in Schemes 1- 7. The groups R, R^{b}, L, and T, are as previously defined unless otherwise noted. It will be readily apparent to one of ordinary skill in the art that other compounds within formulae I, II, and III can be synthesized by substitution of the appropriate reactants and agents in the syntheses shown below. It will also be apparent to one skilled in the art that the selective protection and deprotection steps, as well as the order of the steps themselves, can be carried out in varying order, depending on the nature of groups R, R^{p}, L, and T, in order to successfully complete the syntheses of compounds of formulae I, II, and III.

The conversion of erythromycin A to 1 is described in United States Pat. Nos. 4,990,602; 4,331,803, 4,680,368, and 4,670,549 and European Patent Application EP 260,938.

Briefly, the C-9-carbonyl of erythromycin A can be protected as an oxime. Preferred protecting groups at the C-9-carbonyl are =N-O-R^{x} or =N-O-C(R^{y})(R^{z})(-O-R^{x}), wherein R^{x} is (a) C₁-C₁₂-alkyl, (b) C₁-C₁₂-alkyl substituted with aryl, (c) C₁-C₁₂-alkyl substituted with substituted aryl, (d) C₁-C₁₂-alkyl substituted with heteroaryl, (e) C₁-C₁₂-alkyl substituted with substituted heteroaryl, (f) C₃-C₁₂-cycloalkyl, or (g) -Si-(R^{d})(R^{e})(R^{f}), wherein R^{d}, R^{e} and R^{f} are C₁-C₁₂-alkyl or -Si(aryl)₃, and wherein R^{y} and R^{z} are independently (a) hydrogen, (b) C₁-C₁₂-alkyl, (c) C₁-C₁₂-alkyl substituted with aryl, or (d) C₁-C₁₂-alkyl substituted with substituted aryl, or R^{y} and R^{z} taken together with the carbon to which they are attached form a C₃-C₁₂-cycloalkyl ring. A preferred carbonyl protecting group is O-(1-isopropoxycyclohexyl) oxime.

The 2'- and 4"-hydroxy groups of the C-9 protected erythromycin A can be treated with a hydroxy protecting group precursor in an aprotic solvent. Hydroxy protecting group precursors include, acetic anhydride, benzoic anhydride, benzyl chloroformate, hexamethyldisilazane, or a trialkylsilyl halide: Examples of aprotic solvents include dichloromethane, chloroform, THF, N-methyl pyrrolidinone, DMSO, diethylsulfoxide, DMF, N,N-dimethylacetamide, hexamethylphosphoric triamide, mixtures thereof, and mixtures of one of these solvents with ether, tetrahydrofuran, 1,2-dimethoxyethane, acetonitrile, ethyl acetate, or acetone. Aprotic solvents do not adversely affect the reaction and are preferably dichloromethane, chloroform, DMF, tetrahydrofuran (THF), N-methyl pyrrolidinone, or mixtures thereof. Protection of the 2'- and 4"-hydroxy groups of the C-9 protected erythromycin A may be accomplished sequentially or simultaneously. Preferred protecting groups include acetyl, benzoyl, and trimethylsilyl. An especially preferred protecting group is trimethylsilyl. A thorough discussion of protecting groups and the solvents in which they are most effective is provided in Greene and Wuts in Protective Groups in Organic Synthesis, 2nd ed., John Wiley & Son, Inc., 1991.

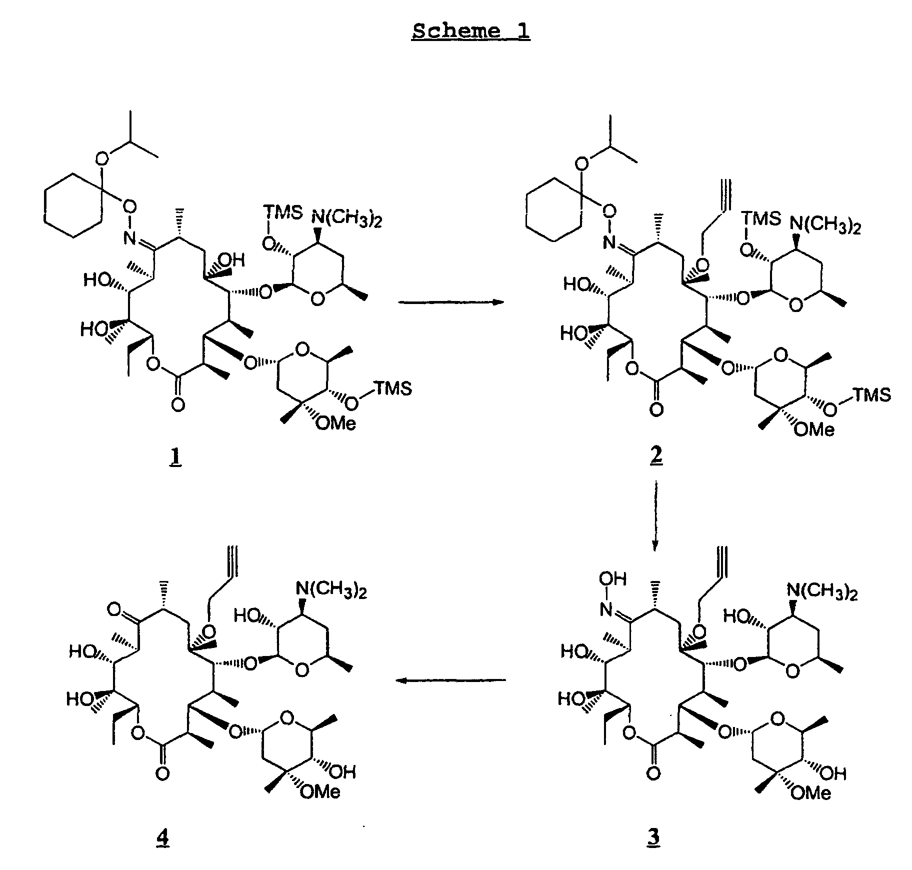

As shown in Scheme 1, conversion of alcohol **1** to ether **2** can be accomplished with an alkylating agent in the presence of base. Alkylating agents include alkyl chlorides, bromides, iodides or alkyl sulfonates. Specific examples of other alkylating agents are allyl bromide, propargyl bromide, benzyl bromide, 2-fluoroethyl bromide, 4-nitrobenzyl bromide, 4-chlorobenzyl bromide, 4-methoxybenzyl bromide, α-bromo-p-tolunitrile, cinnamyl bromide, methyl 4-bromocrotonate, crotyl bromide, 1-bromo-2-pentene, 3-bromo-1-propenyl phenyl sulfone, 3-bromo-1-trimethylsilyl-1-propyne, 3-bromo-2-octyne, 1-bromo-2-butyne, 2-picolyl chloride, 3-picolyl chloride, 4-picolyl chloride, 4-bromomethyl quinoline, bromoacetonitrile, epichlorohydrin, bromofluoromethane, bromonitromethane, methyl bromoacetate, methoxymethyl chloride, bromoacetamide, 2-bromoacetophenone, 1-bromo-2-butanone, bromochloromethane, bromomethyl phenyl sulfone, and 1,3-dibromo-1-propene. Examples of alkyl sulfonates are allyl tosylate, 3-phenylpropyl trifluoromethane sulfonate, and n-butylmethanesulfonate. Examples of the solvents used are aprotic solvents such as (DMSO), diethylsulfoxide, N,N-dimethylformamide, N,N-dimethylacetamide, N-methyl-2-pyrrolidone, hexamethylphosphoric triamide, mixtures thereof or mixtures of one of these solvents with ether, tetrahydrofuran, 1,2-dimethoxyethane, acetonitrile, ethyl acetate, or acetone. Examples of the base which can be used are potassium hydroxide, cesium hydroxide, tetraalkylammonium hydroxide, sodium hydride, potassium hydride, and alkali metal alkoxides such as potassium isopropoxide, potassium *tert*-butoxide, and potassium *iso*butoxide. An especially preferred method of preparing **2** was treatment of alcohol **1** with propargyl bromide in a DMSO/THF mixture with potassium hydroxide as the base. The conversion of **2** to **3** can be accomplished as described in Greene. The preferred conditions for the deprotection of the 2'- and 4"-hydroxyl groups (acetic acid in acetonitrile and water) can result in concomitant removal of the 1-isopropoxycyclohexyl group provide an unalkylated oxime (=N-OH) at C-9. If not, then the conversion can be accomplished in a separate step. The deoximation of **3** to provide Intermediate **4** can be accomplished as described in Greene. Examples of deoximating agents are nitrous acid (formed in situ by the reaction of sodium nitrite with acids such as HCl, H₂SO₄, and TFA) and inorganic sulfur oxide compounds such as sodium hydrogen sulfite, sodium pyrosulfate, sodium thiosulfate, sodium sulfate, sodium sulfite, sodium hydrosulfite, sodium metabisulfite, sodium dithionate, potassium thiosulfate, and potassium metabisulfite in an protic solvent. Examples of protic solvents are water, methanol, ethanol, propanol, isopropanol, trimethylsilanol, or mixtures thereof. The deoximation reaction can also be accomplished with an organic acid such as formic acid, acetic acid and TFA. The amount of acid used is from about 1 to about 10 equivalents per equivalent of **3**. In a preferred embodiment, the deoximation was carried out using sodium nitrite and an inorganic acid such as HCl in ethanol and water to provide the desired 6-O-substituted erythromycin Intermediate **4**, wherein R is propargyl.

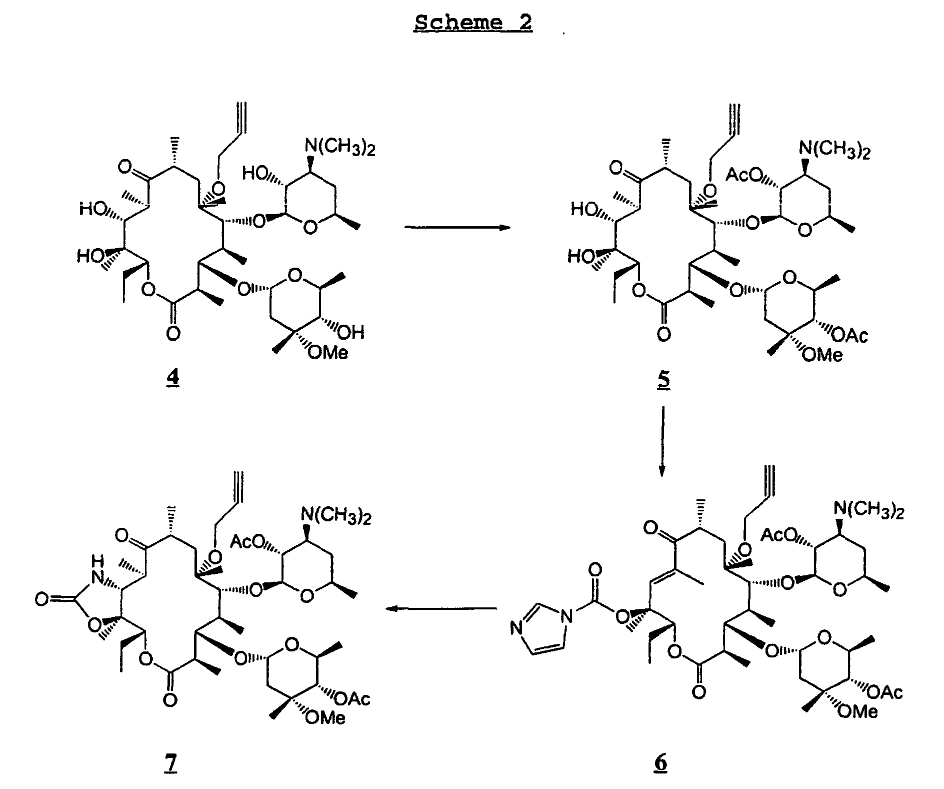

As shown in Scheme 2, conversion of **4** to Intermediate **5** can be accomplished by the 2'- and 4"-hydroxy group protection procedures described previously. Conversion of **5** to **6** can be accomplished with an excess of an alkali metal hydride or bis(trimethylsilyl)amide in the presence of carbonyldiimidazole in an aprotic solvent for about 8 to about 24 hours at temperatures of about -30 °C to about room temperature to provide **6**. The alkali metal may be sodium potassium or lithium and the aprotic solvent can be one defined previously. The reaction may require cooling or heating from about -20 °C to about 70 °C, depending on the conditions used, and preferably from about 0 °C to about room temperature. The reaction requires about 0.5 hours to about 10 days, and preferably about 10 hours to 2 days to complete. Portions of this reaction sequence follow the procedure described by Baker et al., *J. Org. Chem.,* **1988**, 53, 2340 the disclosure of which is herein incorporated by reference. Conversion of **6** to cyclic carbamate **7**, a precursor of compounds of formula **I**, was accomplished by treatment of **6** with liquid ammonia at room temperature for 20 hours.

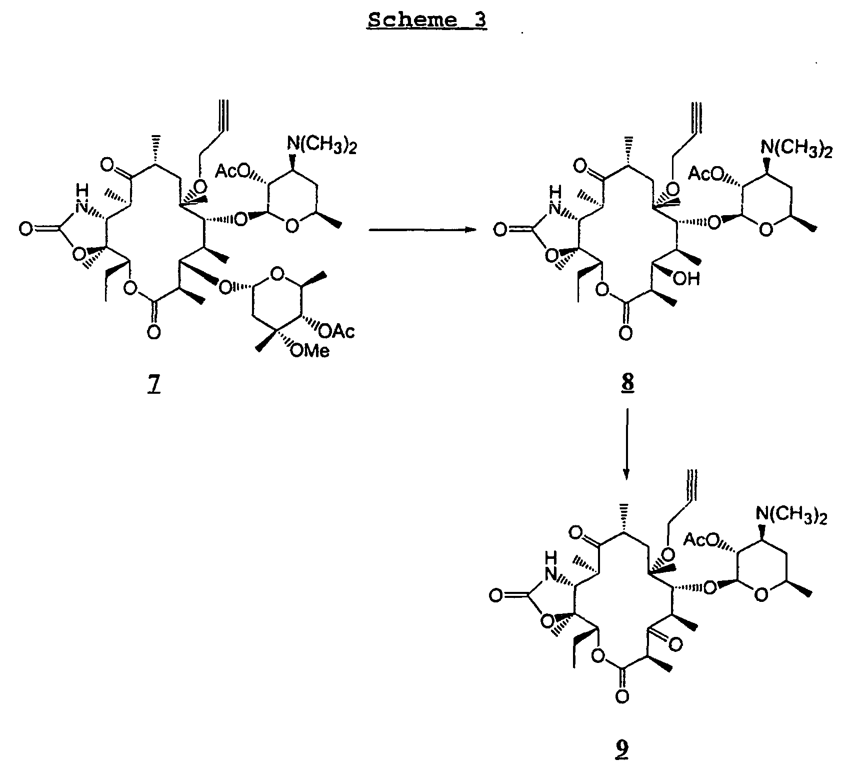

As shown in Scheme 3, **7** can be converted to **8** by hydrolysis of the former with mild aqueous acid or by enzymatic hydrolysis to remove the cladinose moiety from the 3-hydroxy group. Representative acids include dilute hydrochloric acid, sulfuric acid, perchloric acid, chloroacetic acid, dichloroacetic acid, or TFA. Suitable solvents for the reaction include methanol, ethanol, isopropanol, butanol, acetone, and mixtures thereof. Reaction times are typically about 0.5 to about 24 hours. The preferred reaction temperature is about -10 °C to about 60 °C, depending on the method chosen. Alternately, **5** can be treated with acid to remove the protected cladinose group from the 3-hydroxy group as described for the conversion of **7** to **8** and treated with base and carbonyldiimidazole then ammonia as described for the conversion of **5** to **6** and the conversion of **6** to **7**, respectively, to provide **8**. The conversion of **8** to **9** can be accomplished by oxidation of the 3-hydroxy group to a 3-oxo group using a Corey-Kim reaction with N-chlorosuccinimide-dimethyl sulfide or with a modified Swern oxidation procedure using a carbodiimide-DMSO complex. In a preferred method, **8** is added to a preformed N-chlorosuccinimide-dimethyl sulfide complex in a chlorinated solvent such as dichloromethane or chloroform at about -10 to about 25 °C. After stirring for about 0.5 to about 4 hours, a tertiary amine such as triethylamine or diisopropylethylamine is added to produce **9**, a precursor to compounds of formula I.

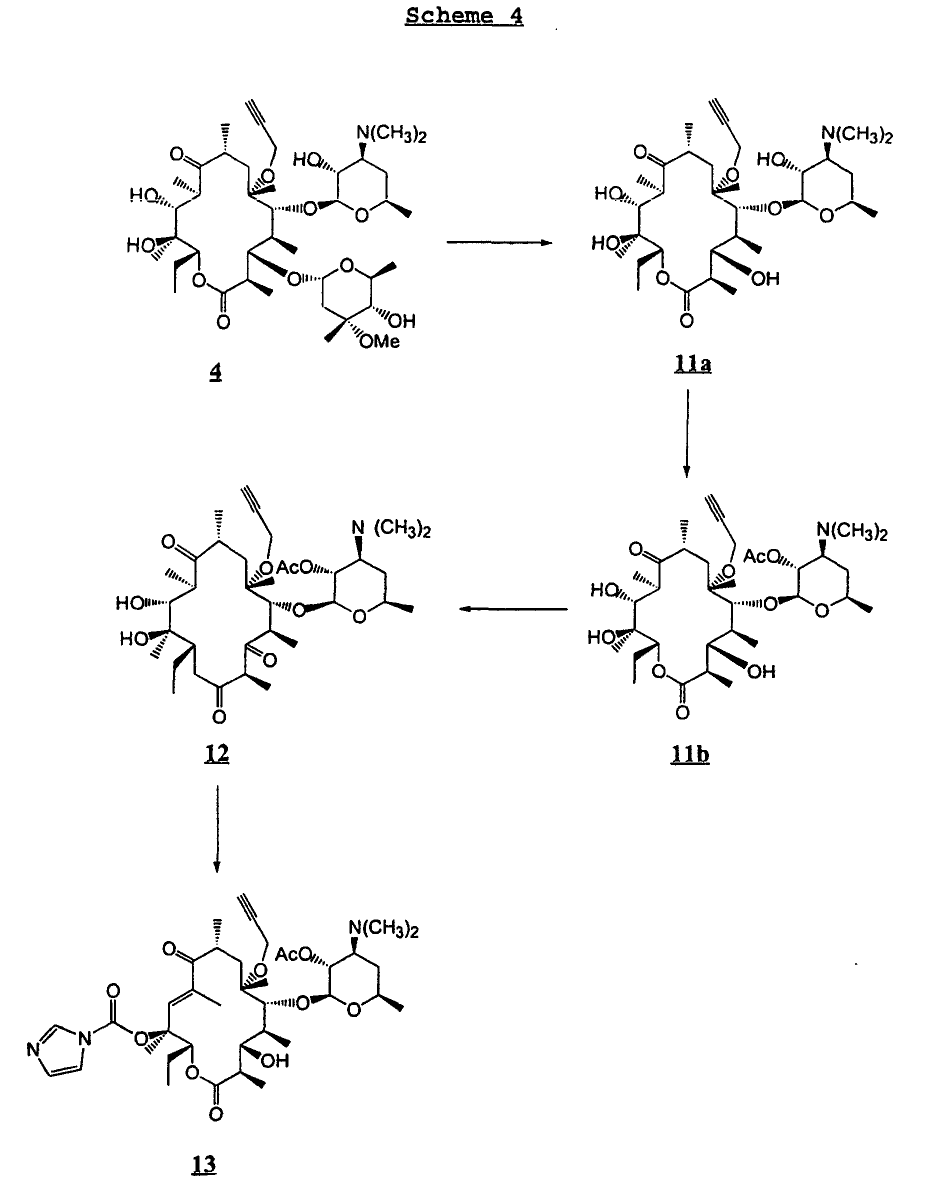

As shown in Scheme 4, **4** (from Scheme 2) can alternatively be (a) treated with acid to remove the cladinose group from the 3-hydroxy group (as described for the conversion of **7** to **8**) to provide **11a**, (b) protected (as described for the conversion of **4** to **5**), to provide **11b**, (c) oxidized (as described for the conversion of **8** to **9**) to provide **12**, and (d) treated with sodium hydride and carbonyldiimidazole (as described for the conversion of **5** to **6**) to provide **13**.

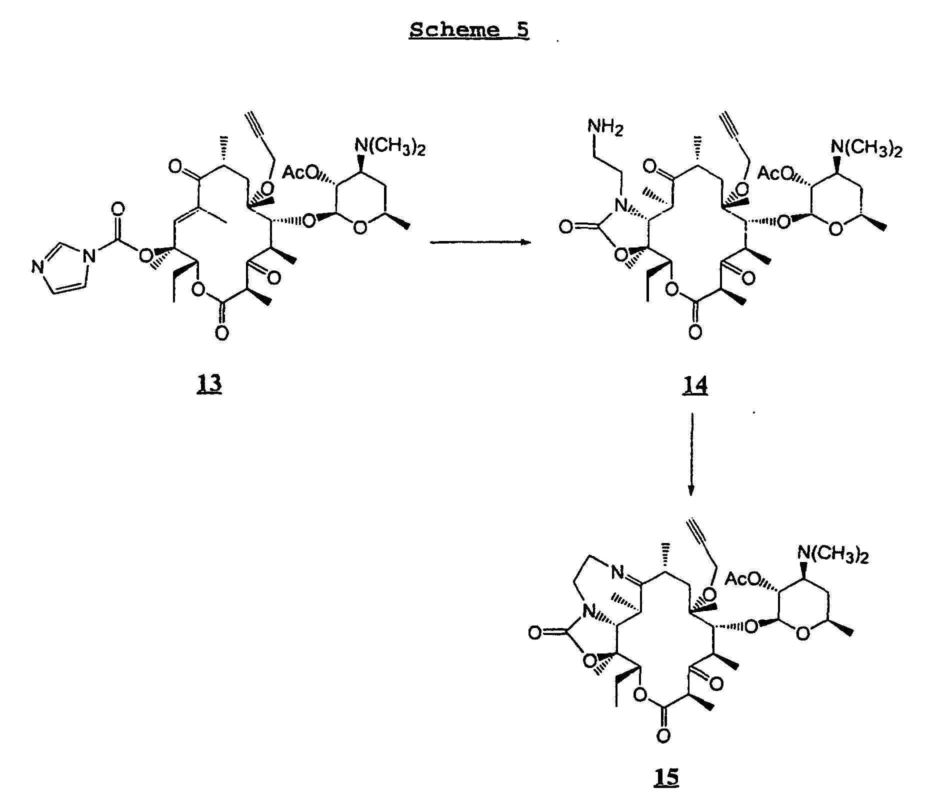

As shown in Scheme 5, **15**, a precursor to compounds of formula II was prepared by treatment of **13** with ethylenediamine in a suitable solvent such as aqueous acetonitrile, DMF, or aqueous DMF to provide **14** which further cyclized by intramolecular condensation with the nearby C-9 carbonyl to form **15**. Preferred conditions for the formation of **15** from **14** are dilute acetic acid or hydrochloric acid in a suitable organic solvent such as ethanol or *iso*-propanol.

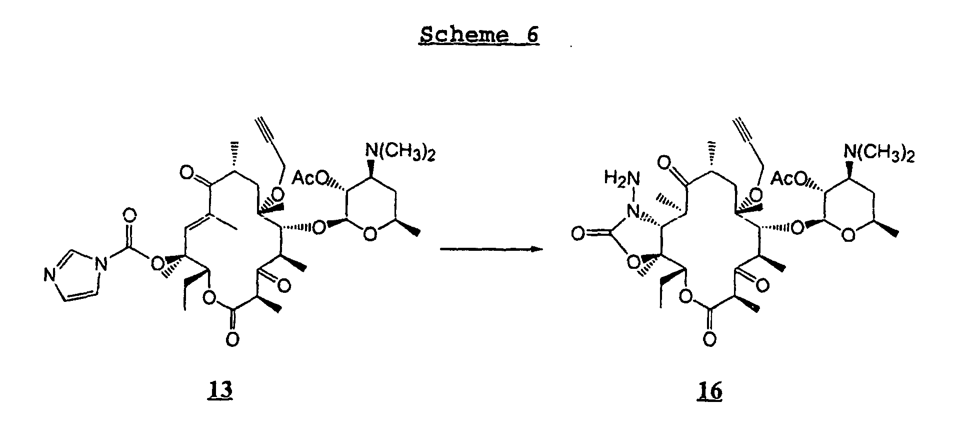

As shown in Scheme 6, **16**, a precursor to compounds of formula III was prepared by treatment of Intermediate **13** with hydrazine in a suitable solvent such as acetonitrile, DMSO, DMF, or mixtures thereof at temperatutes from about room temperature to about 100 °C. In a preferred embodiment, **13** was treated with hydrazine in DMF at a temperature of about 58 °C.

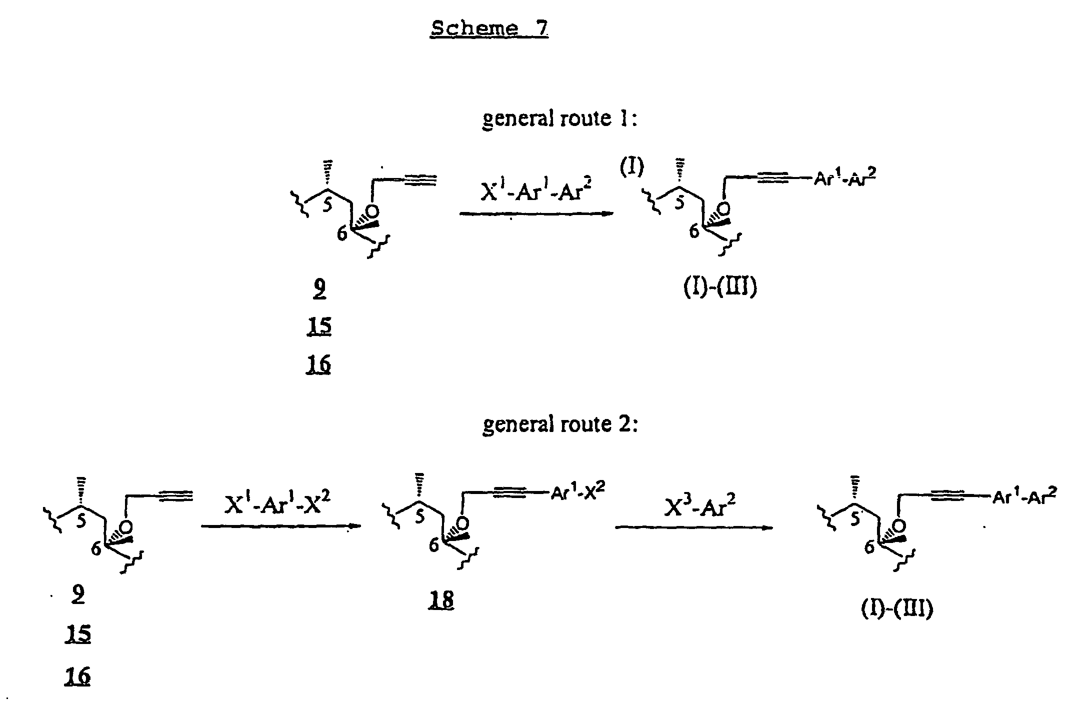

Intermediates **9**, **15**, and **16** can be converted to compounds of formulae I, II, and III, respectively, by a number of general routes. Two preferred general routes are shown in Scheme 7. In general route 1, the 6-O propargyl group of **9**, **15**, or **16** is reacted with R precursor groups such as X¹-Ar¹-Ar², wherein Ar¹ is thienyl, Ar² is pyridyl,
and X¹ is one of any number of covalent bond precursors such as a halide (preferably bromide and iodide) or a sulfonate, to form compounds of formulae I, II, and III. In general route 2, the 6-O propargyl group is reacted with a bifunctionalized aryl or heteroaryl group R precursor, X¹-Ar¹-X², to provide prefunctionalized coupling precursor **18**, wherein X² is halide of sulfonate, which is subsequently coupled to R precursor group X³-Ar², wherein X³ is a covalent bond precursor such as halide (preferably bromide and iodide), sulfonate, trialkylstannane, boronic acid, or borate ester, to provide compounds of formulae I, II, and III. The coupling reactions are performed in the presence of PdII or Pd(0) catalysts with promoters such as phosphines (preferably triphenylphosphine), arsines (preferably triphenylarsine), amines (preferably pyridine and triethylamine), and inorganic bases (preferably potassium carbonate or cesium fluoride) in polar, aprotic solvents such as DMF, DMSO, DME, acetonitrile, THF, or mixtures thereof, at temperatures from about room temperature to about 150 °C, depending on the coupling method chosen and the nature of X¹, X², and X³. A thorough survey of coupling procedures, reagents, and solvents for transition metal-catalyzed couplings is provided in Larock, Comprehensive Organic Transformations. A Guide to Functional Group Preparations, VCH Publishers, New York (1989).

### EXAMPLES

The compounds and processes of the present invention will be better understood in connection with the following examples, which are intended as an illustration of and not a limitation upon the scope of the invention as defined in the appended claims.

### Example 1

### Compound of Formula I: R^{b} is H, L is -C(O)-, T is -NH-, R is -(CH₂)-C≡C-(5-(2-pyridyl)-2-thienyl)

### step 1a: compound 2 from Scheme 1

A stirred solution of compound **1** from Scheme 1 (100 g, 0.0969 mol) in freshly distilled THF (500 mL) and anhydrous DMSO (200 mL) at 0 °C, was treated with propargyl bromide (80 wt. % in toluene, 27 mL, 0.24 mol) followed by dropwise addition over 25 minutes of a solution/slurry of powdered KOH (13.6 g , 0.24 mol) in DMSO (300 mL), stirred vigorously at 0 °C for 1 hour, treated sequentially with powdered KOH (10.9 g, 0.19 mol) and propargyl bromide (80 wt. % in toluene, 0.19 mol), and stirred at 0 °C for 1.5 hours. The addition sequence of KOH (10.9 g) and propargyl bromide solution (21 mL) followed by 1.5 hours of stirring at 0 °C was repeated three times until the reaction was judged to be 60-65% complete by TLC (98:1:1 dichloromethane:methanol:ammonium hydroxide). The cold reaction mixture was diluted with ethyl acetate (1.5 L ) and water (1 L), stirred at 0 °C for 5 minutes, and agitated in a separatory funnel. The resulting organic layer was separated, washed sequentially with water (1L) and brine (2 x 500 mL), dried (MgSO₄), filtered, and concentrated to provide 108 g of a dark brown foam which was used in step 1b without further purification.
MS (FAB) *m*/*z* 1071 (M+H)⁺.

### step 1b: compound 3 from Scheme 1

A suspension of the product from step **1a** (108 g) in acetonitrile (300 mL) was treated sequentially with water (150 mL) and glacial acetic acid (200 mL), stirred at room temperature for 20 hours, and concentrated at 40 °C to provide a brown foam. The foam was dissolved in ethyl acetate (750 mL), washed sequentially with 5% aqueous sodium carbonate (2 x 250 mL) and brine (2 x 250 mL), dried (MgSO₄), filtered, and concentrated to provide 74 g of the crude oxime as a brown foam which was used in step 1c without further purification.

### step 1c: compound 4 from Scheme 1

A solution of the product from step **1b** (74 g) in ethanol (50 mL) was treated sequentially with water (550 mL) and sodium nitrite (33 g, 0.48 mol), stirred at room temperature for 15 minute, treated with 4M HCl (125 mL, 0.48 moles) over 15 minutes, heated to 70 °C for two hours, cooled to room temperature, diluted with ethyl acetate (1.3L), washed sequentially with 5% aqueous sodium carbonate (2 x 350 mL) and brine (2 x 300 mL), dried (MgSO₄), filtered, and concentrated. Purification of the residue by flash chromatography on silica gel with 98:1:1 dichloromethane:methanol:ammonium hydroxide provided 45 g of a yellow foam. Crystallization of the foam from hot acetonitrile provided 27 g of desired product as off-white crystals. MS (APCI) *m*/*z* 772 (M+H)⁺.

### step 1d: compound 5 from Scheme 2

A solution of the product from step **1c** (18.9g, 24.5 mmol) in anhydrous methylene chloride (100 mL) was treated sequentially with 4-(dimethylamino)pyridine (105 mg, 0.86 mmol) and triethylamine (7.16 ml, 51 mmol), cooled to 15-20 °C in a cold water bath, treated with acetic anhydride (5.5 mL, 58 mmol) over 5 minutes, stirred at room temperature for 4.5 hours, diluted with ethyl acetate (300 mL), washed sequentially with 5% aqueous sodium carbonate (2 x 100 mL), water (2 x 100 mL) and brine (2 x 100 mL), dried (MgSO₄), filtered, and concentrated to provide 21 g of the desired product as a white foam which was used without further purification in step 1e.

### step 1e: compound 6 from Scheme 2

A solution of the product from step **1d** (92.38 g, 0.108 mol) in THF (350 mL) and DMF (175 mL) at room temperature was treated with 1,1'-carbonyldiimidazole (61.26 g, 0.378 mol), cooled to 0 °C, treated with sodium hydride (60% dispersion in mineral oil, 5.4 g, 0.135 mol) over 1 hour, stirred an additional 30 minutes at 0 °C and at room temperature for 3 hours, recooled to 0 °C, diluted with ethyl acetate (800 mL), washed sequentially with 5% aqueous sodium bicarbonate (200 mL), water (2 x 500 mL) and brine (2 x 300 mL), dried (Na₂SO₄), filtered, and concentrated to provide 104 g of the desired product as a dark yellow foam which was used without further purification in step If.
MS (ESI) *m*/*z* 932 (M+H)⁺.

### step 1f: compound 7 from Scheme 2

A solution of the product from step **1e** (52 g, 55.8 mmol) in acetonitrile (500 mL) at -78 °C was treated with liquid ammonia (500-600 mL) in a sealed reaction vessel, stirred at room temperature for 24 hours, concentrated first by evaporation of the ammonia at room temperature and atmospheric pressure, and concentrated finally to remove the acetonitrile. The crude product (52 g) was purified by flash chromatography on silica gel with a gradient of from 3:7 acetone/hexanes to 1:1 acetone/hexanes to provide 32 g of the title compound as a yellow foam.

### step 1g: compound 8 from Scheme 3

A suspension of the product from step **1f** (63.92 g, 72.55 mmol) in 1:1 ethanol/water (600 mL) at 0 °C was treated with 4N HCl (393 mL, 1860 mmol) over 20 minutes, stirred at room temperature for 24 hours, recooled to 0. °C, diluted with water (200 mL), adjusted to pH 9-10 with 4N sodium hydroxide solution, diluted with ethyl acetate, washed with brine, dried (Na₂SO₄), filtered, and concentrated to provide 62.1 g of yellow foam. Crystallization of the foam from 1.5:1 ethyl acetate/hexanes (115 mL) provided 34 g of the desired product as a white solid.
MS (ESI) *m*/*z* 681 (M+H)⁺.

### step 1h: compound 9 from Scheme 3

A solution of N-chlorosuccinimide (10.86g, 81.66 mmol) in anhydrous dichloromethane (450 mL) at -10 °C was treated with methyl sulfide (6.98 ml, 95.27 mmol) over 10 minutes, stirred for an additional 10 minutes, treated with a solution of the product from step 1g (37.02 g, 54.44 mmol) in anhydrous dichloromethane (450 mL) over 35 minutes, stirred an additional 25 minutes at -10 °C, treated with triethylamine (7.57 mL, 54.44 mmol) over 10 minutes, stirred at -10 °C for an additional 50 minutes, washed sequentially with saturated. sodium bicarbonate solution and brine, dried (Na₂SO₄), filtered, and concentrated. Purification of the residue by flash chromatography on silica gel with from 3:7 to 1:1 acetone/hexanes provided 31.9 g of the desired product as a pale yellow foam.
MS (ESI) *m*/*z* 679 (M+H)⁺.

### step 1i: option 1: Compound of Formula I: R^{b} is CH₃C(O)-, L is -C(O)-, T is -NH-, R is -(CH₂)-C≡C-(5-iodo-2-thienyl)

A slurry of the product from step 1h (5.02g, 7.40 mmol), 2,5-diiodothiophene (5.47g, 16.29 mmol), dichlorobis(triphenylphosphine)palladium(II) (0.103g, 0.148 mmol), and copper I iodide (0.014g, 0.074 mmol) in triethylamine (18 mL) and acetonitrile (6 mL) was heated at 60 °C for 3 hours, stirred at room temperature for 48 hours, and concentrated. Purification of the residue by flash chromatography on silica gel with from 40:60 to 1:1 acetone-hexanes provided 4.54g (69.4%) of the desired product as a yellow foam.
MS (APCI) *m*/*z* 887 (M+H)⁺.

### step 1i: option 2: Compound of Formula I: R^{b} is CH₃C(O)-, L is -C(O)-, T is -NH-, R is -(CH₂)-C≡C-(5-bromo-2-thienyl)

The product from Example 1h (10.8 g) was processed as described in step 1i, option 1, (substituting 2,5-dibromothiophene for 2,5-diiodothiophene) to provide 8.81 g of the desired product.
MS (APCI) *m*/*z* 841 (M+H)⁺.

### step 1j: Compound of Formula I: R^{b} is CH₃C(O)-,

### L is -C(O)-, T is -NH-, R is -(CH₂)-C≡C-(5-(2-pyridyl)-2-thienyl)

A solution of the product from step 1i, option 2 (300 mg, 0.34 mmol), 2-tri-n-butylstannylpyridine (312 mg, 0.85 mmol), tetrakis(triphenylphosphine)palladium(0) (38 mg, 0.034 mmol) and copperI bromide (2.4 mg, 0.017 mmol) in degassed 1,4-dioxane (2.5 mL) was heated at 90 °C for 21 hours and concentrated. Purification of the residue by flash chromatography on silica gel with from 35:65 to 1:1 acetone-hexanes provided 170 mg of a yellow foam.

### step 1k: Compound of Formula I: R^{b} is H, L is -C(O)-, T is -NH-, R is -(CH₂)-C≡C-(5-(2-pyridyl)-2-thienyl)

A solution of the product from step 1j was dissolved in methanol (10 mL), stirred for 6 hours at room temperature, and concentrated. Purification of the residue by flash chromatography on silica gel with 98:1:1 dichloromethane:methanol:ammonium hydroxide provided 136 mg of the desired product as a yellow foam.
MS (APCI) *m*/*z* 796 (M+H)⁺;
¹³C NMR (100 MHz, CDCl₃) 216.6, 205.1, 169.3, 157.7, 152.1,149.5, 145.8, 136.5, 133.4, 124.5, 124.3, 122.0, 118.9, 103.1, 90.6, 83.5, 79.4, 79.4, 77.3, 77.3, 70.2, 69.5, 65.8, 58.2, 51.7, 51.0, 46.6, 44.7, 40.2, 38.7, 37.3, 28.3, 22.5, 21.1, 19.7, 18.0, 14.7, 14.5, 13.6, 13.6, 10.5;
High Resolution MS (FAB) calcd (M+H)⁺ for C₄₂H₅₈N₃O₁₀S: 796.3843. Found: 796.3826.

### Example 2

### Compound of Formula I: R^{b} is H, L is -C(O)-, T is -NH-, R is -(CH₂)-C≡C-(5-(3-pyridyl)-2-thienyl)

The product from step 1i, option 2 (250 mg), was processed as described in steps 1j and 1k (substituting 3-tri-*n*-butylstannylpyridine for 2-tri-*n*-butylstannylpyridine) to provide 61 mg of the desired product.
High Resolution MS (FAB) calcd (M+H)⁺ for C₄₂H₅₈N₃O₁₀S: 796.3843. Found: 796.3826.

### Example 3

### Compound of Formula I: R^{b} is H, L is -C(O)-, T is -NH-, R is -(CH₂)-C≡C-(5-(4-pyridyl)-2-thienyl)

The product from step 1i, option 2 (350 mg), was processed as described in steps 1j and 1k (substituting 4-tri-*n*-butylstannylpyridine for 2-tri-*n*-butylstannylpyridine) to provide 105 mg of the desired product..
High Resolution MS (ESI) calcd (M+H)⁺ for C₄₂H₅₈N₃O₁₀S: 796.3843. Found: 796.3833.

### Example 4

### Compound of Formula II: R^{b} is H, R is -(CH₂)-C≡C-(5-(2-pyridyl)-2-thienyl)

### step 4a: Compound 11a from Scheme 4

A suspension of compound **4** from Scheme 4 (56 g) in ethanol (180 mL) and water (540 mL) was slowly treated with 1M HCl (130 mL) over 20 minutes, stirred for 7 hours, cooled to 0 °C, treated with 1M NaOH (130 mL), and extracted with ethyl acetate. The extract was washed with brine, dried (MgSO₄), filtered, and concentrated. The residue was redissolved in diethyl ether (300 mL) and extracted into 1M HCl (300 mL). The extract was made basic (pH 10) with 1M NaOH and extracted with ethyl acetate. The extract was washed with brine, dried (MgSO₄), filtered, and concentrated to provide 38 g of the desired product which was used in the next step without further purification.

### step 4b: Compound 11b from Scheme 4

The product from step 4a (37 g) was dissolved in dichloromethane (230 mL) and treated with triethylamine (16.8 mL) followed by dropwise addition of acetic anhydride (11.2 mL) over 10 minutes at room temperature, stirred for 10 hours, diluted with dichloromethane (200 mL), washed sequentially with 5% aqueous NaHCO₃ and brine, dried (MgSO₄), filtered, and concentrated to provide 40 g of the desired product which was used in the next step without further purification.

### step 4c: Compound 12 from Scheme 4

The product from step **4b** (35.73 g) was processed as described in step 1h to provide 40.2 of the crude product which was purified by flash chromatography on silica gel with a gradient of from 98:1:1 dichloromethane/methanol/ammonium hydroxide to 94:5:1 dichloromethane/methanol/ammonium hydroxide to provide 20.5 g of the desired product.

### step 4d: Compound 13 from Scheme 4

A solution of the product from step **4c** (13.57 g, 0.02 mol) in THF (250 mL) 0 °C was treated first with 1,1'-carbonyldiimidazole (16.8 g, 0.103 mol), then portionwise with sodium hydride (60% dispersion in mineral oil, 831 mg) over 20 minutes, stirred at room temperature for 6 days, recooled to 0 °C, diluted with ethyl acetate (500 mL), washed sequentially with 5% aqueous sodium bicarbonate (150 mL), water (2 x 150 mL) and brine (2 x 200 mL), dried (Na₂SO₄), filtered, and concentrated. The residue was purified by flash chromatography on silica gel with a gradient of from 3:7 acetone/hexanes to 4:6 acetone/hexanes provide 5.75 g of the desired product.

### step 4e: Compound 14 from Scheme 5

A slurry of the product from step **4d** (1.5 g, 0.002 mol) in acetonitrile (15 mL) and water (1 mL) was treated with ethylenediamine (1.4 mL), stirred at room temperature for 2 hours, and concentrated. The residue was treated with ethyl acetate, washed with water, dried (Na₂SO₄), filtered and concentrated. The residue was purified by flash chromatography on silica gel with 97.5:2:0.5 dichloromethane:methanol:ammonium hydroxide to provide 0.904 g of the desired product.

### step 4f: Compound 15 from Scheme 5

A solution of the product from step **4e** (0.9 g, 1.25 mmol) in ethanol (25 mL) at room temperature was treated with acetic acid (285 µL), warmed to 78 °C for 18 hours, treated with ethyl acetate (150 mL), washed sequentially with 5% aqueous sodium bicarbonate (75 mL),and brine (2 x 75 mL), dried (Na₂SO₄), filtered, and concentrated. The residue was purified by flash chromatography on silica gel with 8:92 methanol/dichloromethane to provide 0.572 g of the desired product.

### step 4g: Compound of Formula I: R^{b} is H, L is -C(O)-, T is -NH-, R is -(CH₂)-C≡C-(5-(2-benzothiophenyl)-2-thienyl)

The product from step **4f** (380 mg) was processed as described in the reference procedure given below (replacing 2-bromo-5-(4-pyrimidinyl)thiophene with 2-(5-bromo-2-thienyl)pyridine) and purified by flash chromatography on silica gel with a gradient of from 5:95 methanol/dichloromethane to 7:93 methanol/dichloromethane to provide 210 mg of the desired product.
High Resolution MS (FAB) calcd (M+H)⁺ for C₄₄H₆₁N₄O₉S: 821.4154. Found 821.4161.

### Reference procedure for preparing:

### Compound of Formula I: R^{b} is CH₃C(O)-, L is -C(O)-, T is -NH-, R is -(CH₂)-C≡C-(5-(5-pyrimidinyl)-2-thienyl)

A solution of Pd₂(dba)₃ (96 mg, 0.105 mmol) and triphenylarsine (128 mg, 0.42 mmol) in degassed acetonitrile (6 mL) was stirred for 30 minutes, treated sequentially with the product from step 1h (1.04 g, 1.4 mol), 2-bromo-5-(4-pyrimidinyl)thiophene (0.607 g, 2.5 mmol) and copperI iodide (2.7 mg, 0.014 mmol), stirred at 80 °C for 2 hours, cooled, treated with ethyl acetate (50 mL) and water (10 mL), filtered through powdered seashells (Celite®). The water was removed, and the organic layer was washed with brine, dried (Na₂SO₄), filtered, and concentrated.

### Example 5

### Compound of Formula I: R^{b} is H, L is -C(O)-, T is -N(W-R^{d})-, W is -NH-, R^{d} is H, R is -(CH₂)-C≡C-(5-(2-pyridyl)-2-thienyl)

### step 5a: Compound 16 from Scheme 6

A solution of the product from step **4d** (3 g, 4 mmol)in DMF (22 mL) was treated sequentially with 1-hexene (12 mL) and hydrazine (1.29 mL), warmed to 58 °C for 6 hours, treated with saturated aqueous NH₄Cl, and extracted with ethyl acetate. The extract was washed sequentially with 5% aqueous sodium bicarbonate, water, and brine, dried (Na₂SO₄), filtered, and concentrated. The residue (2.2 g) was dissolved in methanol (50 mL), stirred for 20 hours, and concentrated. The residue was purified by flash chromatography on silica gel with 6:3.5:0.5 ethyl acetate/hexanes/ammonium hydroxide to 98.5:2:0.5 dichloromethane/methanol/ammonium hydroxide to provide 1.6 g of the desired product.

### step 5b: Compound of Formula I: R^{b} is H, L is -C(O)-T is -N(W-R^{d})-, W is -NH-, R^{d} is H, R is -(CH₂)-C≡C-(5-(2-pyridyl)-2-thienyl)

A solution of the product from step **5a** (350 mg) was processed as described in step 4g to provide the crude product which was purified by flash chromatography on silica gel with 2:97.5:0.5 methanol/dichloromethane/ammonium hydroxide to provide 229 mg of the desired product.
High Resolution MS (FAB) calcd (M+H)⁺ for C₄₂H₅₉N₄O₁₀S: 811.3946. Found 811.3945.

## Claims

1. A compound selected from the group consisting of a compound of formula I 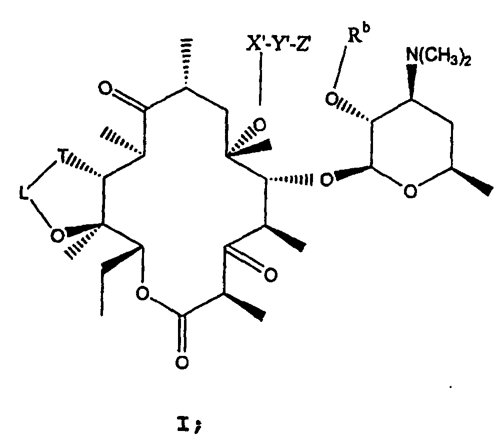 a compound of formula II 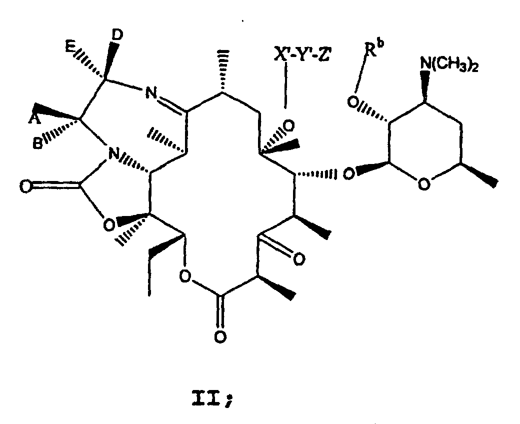 and a compound of formula III 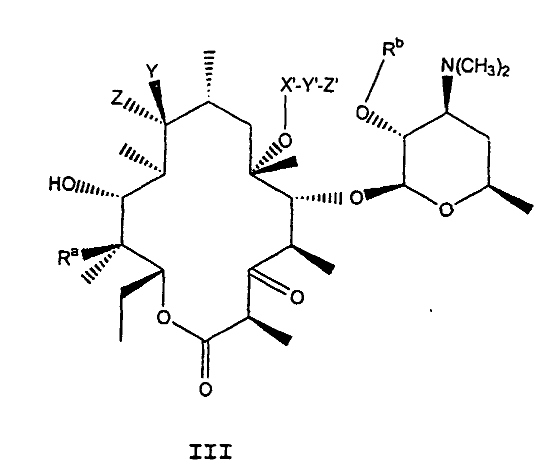 or a pharmaceutically acceptable salt, solvate, ester or prodrug thereof, wherein either:
(a) Y and Z taken together define a group X, and X is selected from the group consisting of
(1) =O,
(2) =N-OH,
(3) =N-O-R¹ where R¹ is selected from the group consisting of
(a) unsubstituted C₁-C₁₂ alkyl,
(b) C₁-C₁₂ alkyl substituted with aryl,
(c) C₁-C₁₂ alkyl substituted with substituted aryl,
(d) C₁-C₁₂ alkyl substituted with heteroaryl,
(e) C₁-C₁₂ alkyl substituted with substituted heteroaryl,
(f) C₃-C₁₂ cycloalkyl, and
(g) -Si- (R²)(R³)(R⁴) wherein R², R³ and R⁴ are each independently selected from C₁-C₁₂ alkyl and aryl;
and
(4) =N-O-C(R⁵) (R⁶)-O-R¹ where R¹ is as previously defined and R⁵ and R⁶ are each independently selected from the group consisting of
(a) hydrogen,
(b) unsubstituted C₁-C₁₂ alkyl,
(c) C₁-C₁₂ alkyl substituted with aryl,
(d) C₁-C₁₂ alkyl substituted with substituted aryl,
(e) C₁-C₁₂ alkyl substituted with heteroaryl,
and
(f) C₁-C₁₂ alkyl substituted with substituted heteroaryl,
or R⁵ and R⁶ taken together with the atom to which they are attached form a C₃-C₁₂ cycloalkyl ring;
or
(b) one of Y and Z is hydrogen and the other is selected from the group consisting of
(1) hydrogen,
(2) hydroxy,
(3) protected hydroxy,
and
(4) NR⁷R⁸ wherein R⁷ and R⁸ are independently selected from hydrogen and C₁-C₆ alkyl, or R⁷ and R⁸ are taken with the nitrogen atom to which they are connected to form a 3- to 7-membered ring which, when the ring is a 5- to 7-membered ring, may optionally contain a hetero function selected from the group consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, and -S- or -S(O)ₙ-, wherein n is 1 or 2;
R^{a} is hydrogen or hydroxy;
R^{b} is hydrogen or a hydroxy protecting group;
L is methylene or carbonyl, provided that when L is methylene, T is -O-;
T is selected from the group consisting of -O-, -NH-, and -N(W-R^{d})-, wherein W is absent or is selected from the group consisting of -O-, -NH-CO-, -N=CH- and -NH-, and R^{d} is selected from the group consisting of
(1) hydrogen,
(2) C₁-C₆ alkyl optionally substituted with one or more substituents selected from the group consisting of
(a) aryl,
(b) substituted aryl,
(c) heteroaryl,
(d) substituted heteroaryl,
(e) hydroxy,
(f) C₁-C₆ alkoxy,
(g) NR⁷R⁸, wherein R⁷ and R⁸ are as defined previously,
and
(h) -CH₂-M-R⁹,
wherein M is selected from the group consisting of
(i) -C(O)-NH-,
(ii) -NH-C(O)-,
(iii) -NH-,
(iv) -N=,
(v) -N(CH₃)-,
(vi) -NH-C(O)-O-
(vii) -NH-C(O)-NH-
(viii) -O-C(O)-NH-
(ix) -O-C(O)-O-
(x) -O-,
(xi) -S(O)ₙ-, wherein n is 0, 1 or 2,
(xii) -C (O)-O-,
(xiii) -O-C(O)-, and
(xiv) -C(O)-;
and
wherein R⁹ is selected from the group consisting of:
(i) C₁-C₆ alkyl, optionally substituted with a substituent selected from the group consisting of
(aa) aryl,
(bb) substituted aryl,
(cc) heteroaryl, and
(dd) substituted heteroaryl;
(ii) aryl,
(iii) substituted aryl,
(iv) heteroaryl,
(v) substituted heteroaryl,
and
(vi) heterocycloalkyl;
(3) C₃-C₇ cycloalkyl,
(4) aryl,
(5) substituted aryl,
(6) heteroaryl,
and
(7) substituted heteroaryl;
X' is C₃ alkynyl;
Y' is thienyl;
Z' is pyridyl;
and A, B, D and E are independently selected from the group consisting of:
(a) hydrogen;
(b) C₁-C₆ alkyl, optionally substituted with one or more substituents selected from the group consisting of:
(i) aryl;
(ii) substituted aryl;
(iii) heteroaryl;
(iv) substituted heteroaryl;
(v) heterocycloalkyl;
(vi) hydroxy;
(vii) C₁-C₆ alkoxy;
(viii) halogen consisting of Br, Cl, F or I; and
(ix) NR⁷R⁸, wherein R⁷ and R⁸ are as previously defined;
(c) C₃-C₇ cycloalkyl;
(d) aryl;
(e) substituted aryl;
(f) heteroaryl;
(g) substituted heteroaryl;
(h) heterocycloalkyl; and
(i) a group selected from option (b) above further substituted with -M-R⁹, wherein M and R⁹ are as previously defined, with the proviso that at least two of A, B, D and E are hydrogen,;
or any one pair of substituents, consisting of AB, AD, AE, BD, BE or DE, is taken together with the atom or atoms to which they are attached to form a 3- to 7-membered ring optionally containing a hetero function selected from the group consisting of -O-, -NH-, -N(C₁-C₆ alkyl-)-, -N(aryl-C₁-C₆ alkyl-)-, -N(substituted aryl-C₁-C₆ alkyl-) -, -N(heteroaryl-C₁-C₆ alkyl-)-, -N(substituted heteroaryl- C₁-C₆ alkyl-)-, -S- or -S(O)ₙ-, wherein n is 1 or 2, -C(O)-NH, -C(O)-NR¹²-, wherein R¹² is selected from the group consisting of hydrogen, C₁-C₃ alkyl, C₁-C₃ alkyl substituted with aryl, substituted aryl, heteroaryl, or substituted heteroaryl, -NH-C(O)-, and -NR¹²-C(O)-.

2. A compound according to Claim 1 wherein L is carbonyl.

3. A compound according to Claim 1 wherein T is -NH-.

4. A compound according to Claim 1 wherein R^{b} is a hydroxy protecting group.

5. A compound according to Claim 1 wherein R^{b} is hydrogen.

6. A compound according to Claim 1, wherein -X', Y', and Z' combine to form a group R, and R is selected from the group consisting of
-(CH₂)-C≡C-(5-(2-pyridyl)-2-thienyl),
-(CH₂)-C≡C-(5-(3-pyridyl)-2-thienyl), and
-(CH₂)-C≡C-(5-(4-pyridyl)-2-thienyl).

7. A compound according to Claim 1 of formula I wherein R^{b} is H, L is -C(O)-, T is -NH-, R is -(CH₂)-C≡C-(5-(2-pyridyl)-2-thienyl).

8. A pharmaceutical composition comprising a pharmaceutically effective amount of a compound of Claim 1 in combination with a pharmaceutically acceptable carrier.

9. A pharmaceutical composition comprising a pharmaceutically effective amount of a compound of Claim 7 in combination with a pharmaceutically acceptable carrier.

10. Use of a compound of Claim 1 for manufacturing a medicament for treating a bacterial infection in a mammal in need of such treatment.

11. Use of a compound of Claim 7 for manufacturing a medicament for treating a bacterial infection in a mammal in need of such treatment.

12. A compound of Claim 1 for use as a therapeutic agent.

13. A process for preparing a compound selected from the group consisting of a compound of formula I 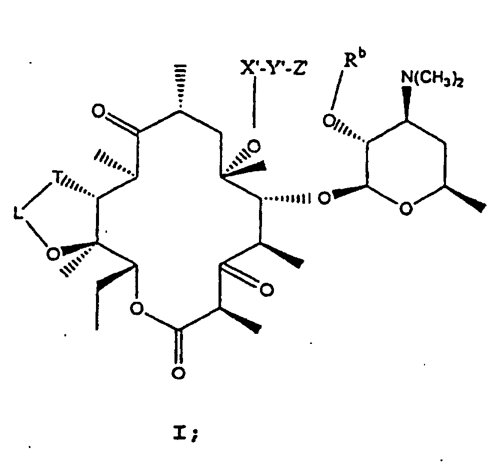 a compound of formula II 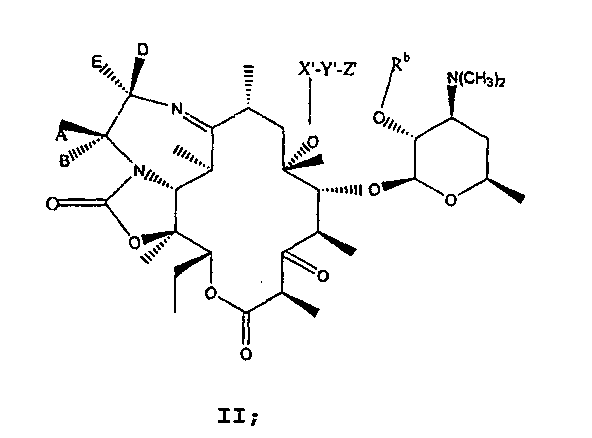 and a compound of formula III 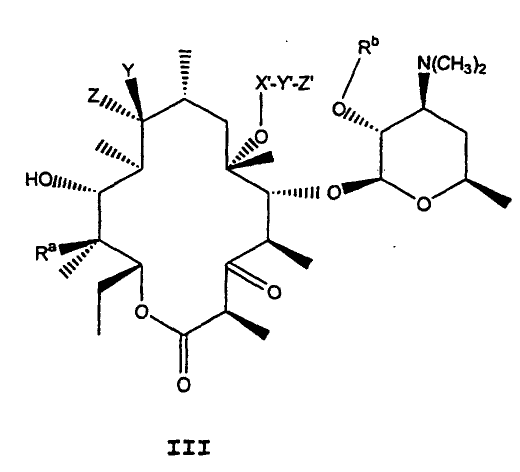 or a pharmaceutically acceptable salt, solvate, ester or prodrug thereof, wherein either:
(a) Y and Z taken together define a group X, and X is selected from the group consisting of
(1) =O,
(2) =N-OH,
(3) =N-O-R¹ where R¹ is selected from the group consisting of
(a) unsubstituted C₁-C₁₂ alkyl,
(b) C₁-C₁₂ alkyl substituted with aryl,
(c) C₁-C₁₂ alkyl substituted with substituted aryl,
(d) C₁-C₁₂ alkyl substituted with heteroaryl,
(e) C₁-C₁₂ alkyl substituted with substituted heteroaryl,
(f) C₃-C₁₂ cycloalkyl, and
(g) -Si-(R²)(R³)(R⁴) wherein R², R³ and R⁴ are each independently selected from C₁-C₁₂ alkyl and aryl;
and
(4) =N-O-C(R⁵) (R⁶)-O-R¹ where R¹ is as previously defined and R⁵ and R⁶ are each independently selected from the group consisting of
(a) hydrogen,
(b) unsubstituted C₁-C₁₂ alkyl,
(c) C₁-C₁₂ alkyl substituted with aryl,
(d) C₁-C₁₂ alkyl substituted with substituted aryl,
(e) C₁-C₁₂ alkyl substituted with heteroaryl,
and
(f) C₁-C₁₂ alkyl substituted with substituted heteroaryl,
or R⁵ and R⁶ taken together with the atom to which they are attached form a C₃-C₁₂ cycloalkyl ring;
or
(b) one of Y and Z is hydrogen and the other is selected from the group consisting of
(1) hydrogen,
(2) hydroxy,
(3) protected hydroxy,
and
(4) NR⁷R⁸ wherein R⁷ and R⁸ are independently selected from hydrogen and C₁-C₆ alkyl, or R⁷ and R⁸ are taken with the nitrogen atom to which they are connected to form a 3- to 7-membered ring which, when the ring is a 5- to 7-membered ring, may optionally contain a hetero function selected from the group consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, and -S- or -S(O)ₙ-, wherein n is 1 or 2;
R^{a} is hydrogen or hydroxy;
R^{b} is hydrogen or a hydroxy protecting group;
L is methylene or carbonyl, provided that when L is methylene, T is -O-;
T is selected from the group consisting of -O-, -NH-, and -N(W-R^{d})-, wherein W is absent or is selected from the group consisting of -O-, -NH-CO-, -N=CH- and -NH-, and R^{d} is selected from the group consisting of
(1) hydrogen,
(2) C₁-C₆ alkyl optionally substituted with one or more substituents selected from the group consisting of
(a) aryl,
(b) substituted aryl,
(c) heteroaryl,
(d) substituted heteroaryl,
(e) hydroxy,
(f) C₁-C₆ alkoxy,
(g) NR⁷R⁸, wherein R⁷ and R⁸ are as defined previously,
and
(h) -CH₂-M-R⁹,
wherein M is selected from the group consisting of
(i) -C(O)-NH-,
(ii) -NH-C(O)-,
(iii) -NH-,
(iv) -N=,
(v) -N(CH₃)-,
(vi) -NH-C(O)-O-
(vii) -NH-C(O)-NH-
(viii) -O-C(O)-NH-
(ix) -O-C(O)-O-
(x) -O-,
(xi) -S(O)ₙ-, wherein n is 0, 1 or 2,
(xii) -C(O)-O-,
(xiii) -O-C(O)-,
and
(xiv) -C(O)-;
and
wherein R⁹ is selected from the group consisting of:
(i) C₁-C₆ alkyl, optionally substituted with a substituent selected from the group consisting of
(aa) aryl,
(bb) substituted aryl,
(cc) heteroaryl, and
(dd) substituted heteroaryl;
(ii) aryl,
(iii) substituted aryl,
(iv) heteroaryl,
(v) substituted heteroaryl,
and
(vi) heterocycloalkyl;
(3) C₃-C₇ cycloalkyl,
(4) aryl,
(5) substituted aryl,
(6) heteroaryl,
and
(7) substituted heteroaryl;
X' is
C₃ alkynyl;
Y' is thienyl;
Z' is pyridyl; and A, B, D and E are independently selected from the group consisting of:
(a) hydrogen;
(b) C₁-C₆ alkyl, optionally substituted with one or more substituents selected from the group consisting of:
(i) aryl;
(ii) substituted aryl;
(iii) heteroaryl;
(iv) substituted heteroaryl;
(v) heterocycloalkyl;
(vi) hydroxy;
(vii) C₁-C₆ alkoxy;
(viii) halogen consisting of Br, Cl, F or I; and
(ix) NR⁷R⁸, wherein R⁷ and R⁸ are as previously defined;
(c) C₃-C₇ cycloalkyl;
(d) aryl;
(e) substituted aryl;
(f) heteroaryl;
(g) substituted heteroaryl;
(h) heterocycloalkyl; and
(i) a group selected from option (b) above further substituted with -M-R⁹, wherein M and R⁹ are as previously defined, with the proviso that at least two of A, B, D and E are hydrogen,;
or any one pair of substituents, consisting of AB, AD, AE, BD, BE or DE, is taken together with the atom or atoms to which they are attached to form a 3- to 7-membered ring optionally containing a hetero function selected from the group consisting of -O-, -NH-, -N(C₁-C₆ alkyl-)-, -N(aryl-C₁-C₆ alkyl-)-, -N(substituted aryl-C₁-C₆ alkyl-)-, -N(heteroaryl-C₁-C₆ alkyl-)-, -N(substituted heteroaryl- C₁-C₆ alkyl-)-, -S- or -S(O)ₙ-,
wherein n is 1 or 2, -C(O)-NH, -C(O)-NR¹²-, wherein R¹² is selected from the group consisting of hydrogen, C₁-C₃ alkyl, C₁-C₃ alkyl substituted with aryl, substituted aryl, heteroaryl, or substituted heteroaryl, -NH-C(O)-, and -NR¹²-C(O)-;
wherein said process comprises:
coupling a compound selected from the group consisting of a compound of formula I^{a} 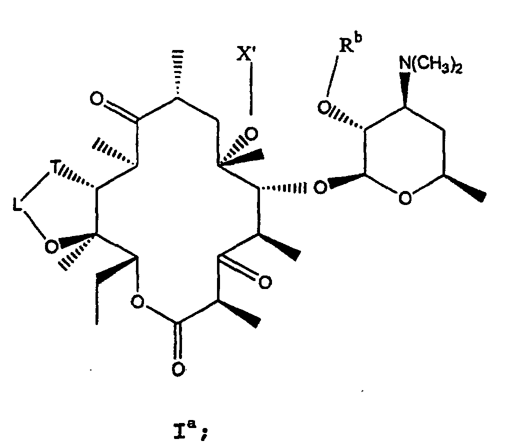
a compound of formula II^{a} 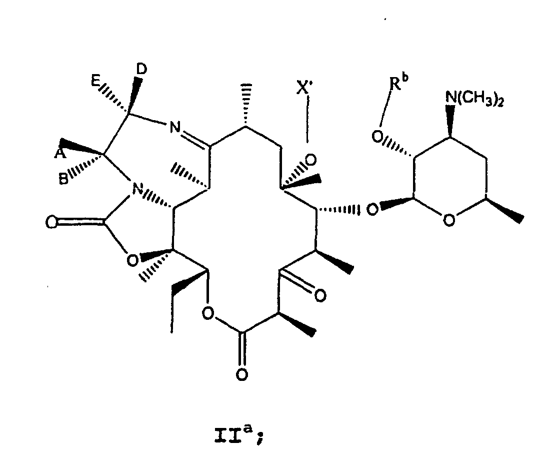
and a compound of formula III^{a} 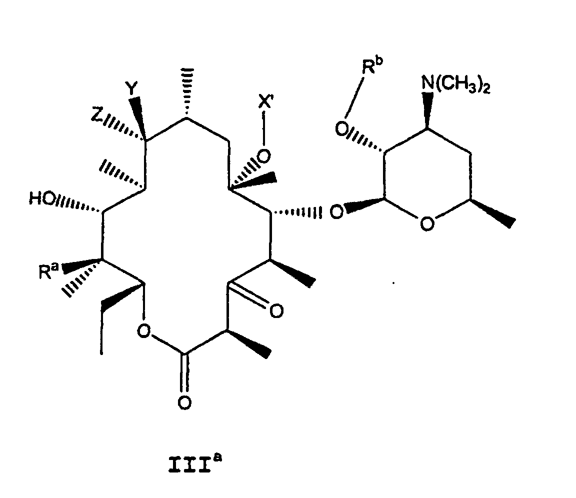
wherein
R^{b} is a hydroxy protecting group, and
Y, Z, X, R¹, R², R³, R⁴, R⁵, R⁶, R⁷, R⁸, n, R^{a}, L, T, R^{d}, W,
M, R⁹, X', Y' , Z', A, B, D, E, R¹² are as previously defined;
with a compound selected from the group consisting of X¹-Y'-Z' and X¹-Y'-X², wherein X¹ and X² are independently selected from the group consisting of bromide, iodide, sulfonate, trialkylstannane, boronic acid, and borate, and Y' and Z' are as previously defined;
in the presence of a palladium catalyst,
with the proviso that when X¹-Y'-X² is employed, a subsequent coupling reaction is performed with X³-Z',
wherein X³ is selected from the group consisting of bromide, iodide, sulfonate, trialkylstannane, boronic acid, and borate, and Z' is as previously defined;
(b) optionally deprotecting;
and
(c) optionally isolating the desired compound.

## Patentansprüche

1. Eine Verbindung gewählt aus der Gruppe bestehend aus einer Verbindung von Formel I 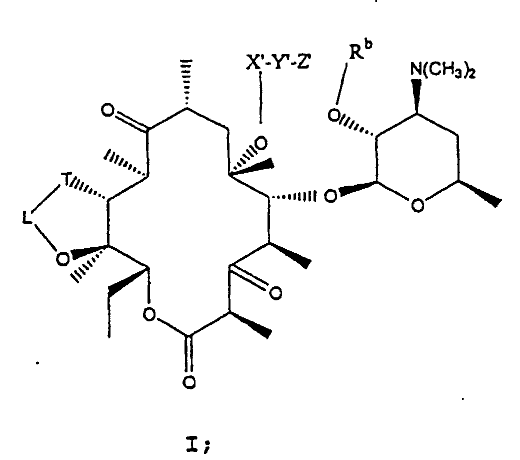 einer Verbindung von Formel II 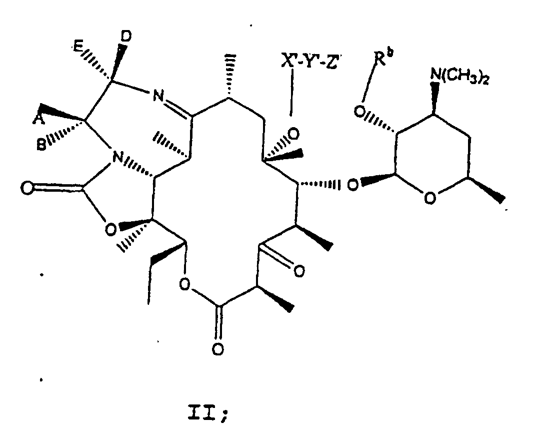 und einer Verbindung von Formel III 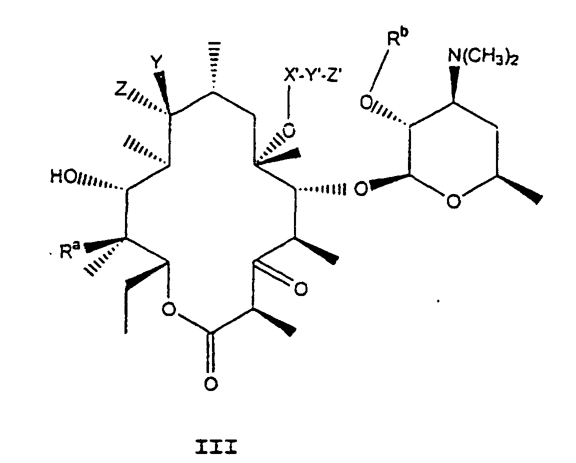 oder einem pharmazeutisch verträglichen Salz, Solvat, Ester oder Prodrug davon, worin jeweils:
(a) Y und Z zusammengenommen eine Gruppe X definieren, und X gewählt ist aus der Gruppe bestehend aus
(1) =O,
(2) =N-OH,
(3) =N-O-R¹, worin R¹ gewählt ist aus der Gruppe bestehend aus
(a) unsubstituiertem C₁-C₁₂ Alkyl,
(b) C₁-C₁₂ Alkyl substituiert mit Aryl,
(c) C₁-C₁₂ Alkyl substituiert mit substituiertem Aryl,
(d) C₁-C₁₂ Alkyl substituiert mit Heteroaryl,
(e) C₁-C₁₂ Alkyl substituiert mit substituiertem Heteroaryl,
(f) C₃-C₁₂ Cycloalkyl, und
(g) -Si-(R²)(R³)(R⁴), worin R², R³ und R⁴ jeweils unabhängig gewählt sind aus C₁-C₁₂ Alkyl und Aryl;
und
(4) =N-O-C(R⁵)(R⁶)-O-R¹, worin R¹ wie vorher definiert ist und R⁵ und R⁶ jeweils unabhängig gewählt sind aus der Gruppe bestehend aus
(a) Wasserstoff,
(b) unsubstituiertem C₁-C₁₂ Alkyl,
(c) C₁-C₁₂ Alkyl substituiert mit Aryl,
(d) C₁-C₁₂ Alkyl substituiert mit substituiertem Aryl,
(e) C₁-C₁₂ Alkyl substituiert mit Heteroaryl, und
(f) C₁-C₁₂ Alkyl substituiert mit substituiertem Heteroaryl,
oder R⁵ und R⁶ zusammengenommen mit dem Atom, an welches sie gebunden sind, bilden einen C₃-C₁₂ Cycloalkylring;
oder
(b) eins von Y und Z ist Wasserstoff und das andere ist gewählt aus der Gruppe bestehend aus
(1) Wasserstoff,
(2) Hydroxy,
(3) geschütztem Hydroxy,
und
(4) NR⁷R⁸, worin R⁷ und R⁸ unabhängig gewählt sind aus Wasserstoff und C₁-C₆ Alkyl, oder R⁷ und R⁸ bilden zusammen mit dem Stickstoffatom, an welches sie gebunden sind, einen 3- bis 7-gliedrigen Ring, der, wenn der Ring ein 5- bis 7-gliedriger Ring ist, wahlweise eine Heterofunktion enthalten kann, gewählt aus der Gruppe bestehend aus -O-, -NH-, -N(C₁-C₆-Alkyl-)-, -N(Aryl)-, -N(Aryl-C₁-C₆-alkyl-)-, -N(substituiertes-Aryl-C₁-C₆alkyl-)-, -N(Heteroaryl)-, -N(Heteroaryl-C₁-C₆-alkyl-)-, -N(substituiertes-Heteroaryl-C₁-C₆-alkyl-)- und -S- oder -S(O)ₙ-, worin n 1 oder 2 ist;
R^{a} ist Wasserstoff oder Hydroxy;
R^{b} ist Wasserstoff oder eine Hydroxyschutzgruppe;
L ist Methylen oder Carbonyl, vorausgesetzt, daß, wenn L Methylen ist, T -O- ist;
T ist gewählt aus der Gruppe bestehend aus -O-, -NH-, und -N(W-R^{d})-, worin W abwesend ist oder gewählt ist aus der Gruppe bestehend aus -O-, -NH-CO-, -N=CH- und -NH-, und R^{d} ist gewählt aus der Gruppe bestehend aus
(1) Wasserstoff,
(2) C₁-C₆ Alkyl wahlweise substituiert mit einem oder mehreren Substituenten gewählt aus der Gruppe bestehend aus
(a) Aryl,
(b) substituiertem Aryl,
(c) Heteroaryl,
(d) substituiertem Heteroaryl,
(e) Hydroxy,
(f) C₁-C₆ Alkoxy,
(g) NR⁷R⁸, worin R⁷ und R⁸ wie vorher definiert sind,
und
(h) -CH₂-M-R⁹,
worin M gewählt ist aus der Gruppe bestehend aus
(i) -C(O)-NH-,
(ii) -NH-C(O)-,
(iii) -NH-,
(iv) -N=,
(v) -N(CH₃)-,
(vi) -NH-C(O)-O-,
(vii) -NH-C(O)-NH-,
(viii) -O-C(O)-NH-,
(ix) -O-C(O)-O-,
(x) -O-,
(xi) -S(O)ₙ-, worin n 0, 1 oder 2 ist,
(xii) -C(O)-O-,
(xiii) -O-C(O)-,
und
(xiv) -C(O)-;
und
worin R⁹ gewählt ist aus der Gruppe bestehend aus:
(i) C₁-C₆ Alkyl, wahlweise substituiert mit einem Substituenten gewählt aus der Gruppe bestehend aus
(aa) Aryl,
(bb) substituiertem Aryl,
(cc) Heteroaryl, und
(dd) substituiertem Heteraryl;
(ii) Aryl,
(iii) substituiertem Aryl,
(iv) Heteroaryl,
(v) substituiertem Heteroaryl,
und
(vi) Heterocycloalkyl;
(3) C₃-C₇ Cycloalkyl,
(4) Aryl,
(5) substituiertem Aryl,
(6) Heteroaryl,
und
(7) substituiertem Heteroaryl;
X' ist C₃ Alkinyl;
Y' ist Thienyl;
Z' ist Pyridyl;
und A, B, D und E sind unabhängig gewählt aus der Gruppe bestehend aus:
(a) Wasserstoff;
(b) C₁-C₆ Alkyl, wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Heterocycloalkyl;
(vi) Hydroxy;
(vii) C₁-C₆ Alkoxy;
(viii) Halogen bestehend aus Br, Cl, F oder I;
und
(ix) NR⁷R⁸, worin R⁷ und R⁸ wie vorher definiert sind;
(c) C₃-C₇ Cycloalkyl;
(d) Aryl;
(e) substituiertes Aryl;
(f) Heteroaryl;
(g) substituiertes Heteroaryl;
(h) Heterocycloalkyl; und
(i) eine Gruppe gewählt aus Option (b) oben, weiter substituiert mit -M-R⁹, worin M und R⁹
wie vorher definiert sind, unter der Voraussetzung, daß mindestens zwei von A, B, D und E Wasserstoff sind;
oder irgendein Paar von Substituenten, bestehend aus AB, AD, AE, BD, BE oder DE, werden zusammengenommen mit dem Atom oder den Atomen, an welche sie gebunden sind, um einen 3- bis 7-gliedrigen Ring zu bilden, wahlweise enthaltend eine Heterofunktion gewählt aus der Gruppe bestehend aus -O-, -NH-, -N(C₁-C₆ Alkyl-)-, -N(Aryl-C₁-C₆ alkyl-)-, -N(substituiertes Aryl-C₁-C₆ Alkyl-)-, -N(Heteroaryl-C₁-C₆ Alkyl-)-, -N(substituiertes Heteroaryl-C₁-C₆ Alkyl-)-, -S- oder -S(O)ₙ-, worin n 1 oder 2 ist, -C(O)-NH-, -C(O)-NR¹²-, worin R¹² gewählt ist aus der Gruppe bestehend aus Wasserstoff, C₁-C₃ Alkyl, C₁-C₃ Alkyl substituiert mit Aryl, substituiertem Aryl, Heteroaryl oder substituiertem Heteroaryl, -NH-C(O)-, und -NR¹²-C(O)-.

2. Eine Verbindung gemäß Anspruch 1, worin L Carbonyl ist.

3. Eine Verbindung gemäß Anspruch 1, worin T -NH- ist.

4. Eine Verbindung gemäß Anspruch 1, worin R^{b} eine Hydroxyschutzgruppe ist.

5. Eine Verbindung gemäß Anspruch 1, worin R^{b} Wasserstoff ist.

6. Eine Verbindung gemäß Anspruch 1, worin sich -X', Y' und Z' verbinden, um eine Gruppe R zu bilden, und R ist gewählt aus der Gruppe bestehend aus
-(CH₂)-C≡C-(5-(2-Pyridyl)-2-thienyl),
-(CH₂)-C≡C-(5-(3-Pyridyl)-2-thienyl), und
-(CH₂)-C≡C-(5-(4-Pyridyl)-2-thienyl).

7. Eine Verbindung gemäß Anspruch 1 von Formel I, worin R^{b} H ist, L -C(O)- ist, T -NH- ist, R -(CH₂)-C≡C-(5-(2-Pyridyl)-2-thienyl) ist.

8. Eine pharmazeutische Zusammensetzung, die eine pharmazeutisch wirksame Menge einer Verbindung von Anspruch 1 in Kombination mit einem pharmazeutisch verträglichen Träger umfaßt.

9. Eine pharmazeutische Zusammensetzung, die eine pharmazeutisch wirksame Menge einer Verbindung von Anspruch 7 in Kombination mit einem pharmazeutisch verträglichen Träger umfaßt.

10. Verwendung einer Verbindung von Anspruch 1 zur Herstellung eines Medikaments zur Behandlung einer bakteriellen Infektion in einem Säugtier, das eine solche Behandlung benötigt.

11. Verwendung einer Verbindung von Anspruch 7 zur Herstellung eines Medikaments zur Behandlung einer bakteriellen Infektion in einem Säugetier, das eine solche Behandlung benötigt.

12. Eine Verbindung von Anspruch 1 zur Verwendung als ein therapeutischer Wirkstoff.

13. Ein Verfahren zur Herstellung einer Verbindung, die gewählt ist aus der Gruppe bestehend aus einer Verbindung von Formel I 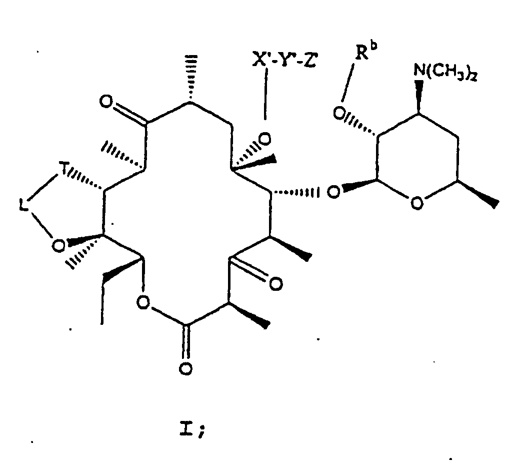 einer Verbindung von Formel II 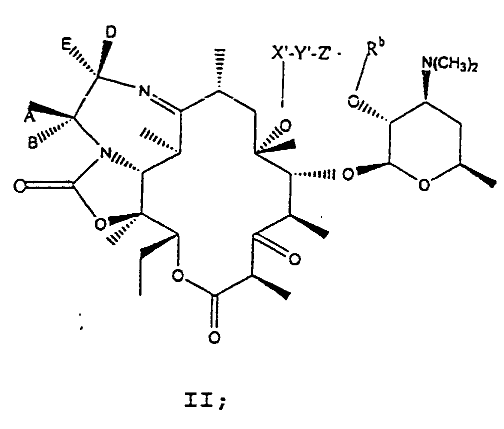 und einer Verbindung von Formel III 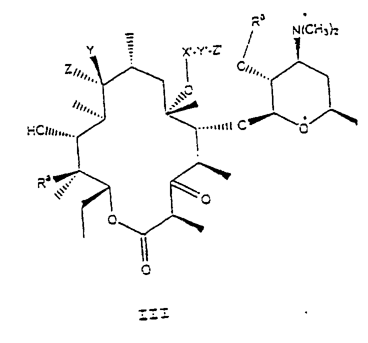 oder einem pharmazeutisch verträglichen Salz, Solvat, Ester oder Prodrug davon, worin entweder:
(a) Y und Z zusammengenommen eine Gruppe X definieren, und X ist gewählt aus der Gruppe bestehend aus
(1) =O,
(2) =N-OH,
(3) =N-O-R¹, worin R¹ gewählt ist aus der Gruppe bestehend aus
(a) unsubstituiertem C₁-C₁₂ Alkyl,
(b) C₁-C₁₂ Alkyl substituiert mit Aryl,
(c) C₁-C₁₂ Alkyl substituiert mit substituiertem Aryl,
(d) C₁-C₁₂ Alkyl substituiert mit Heteroaryl,
(e) C₁-C₁₂ Alkyl substituiert mit substituiertem Heteroaryl,
(f) C₃-C₁₂ Cycloalkyl, und
(g) -Si-(R²)(R³)(R⁴), worin R², R³ und R⁴ jeweils unabhängig gewählt sind aus C₁-C₁₂ Alkyl und Aryl;
und
(4) =N-O-C(R⁵)(R⁶)-O-R¹, worin R¹ wie vorher definiert ist, und R⁵ und R⁶ sind jeweils unabhängig gewählt aus der Gruppe bestehend aus
(a) Wasserstoff,
(b) unsubstituiertem C₁-C₁₂ Alkyl,
(c) C₁-C₁₂ Alkyl substituiert mit Aryl,
(d) C₁-C₁₂ Alkyl substituiert mit substituiertem Aryl,
(e) C₁-C₁₂ Alkyl substituiert mit Heteroaryl, und
(f) C₁-C₁₂ Alkyl substituiert mit substituiertem Heteroaryl,
oder R⁵ und R⁶ zusammengenommen mit dem Atom, an welches sie gebunden sind, bilden einen C₃-C₁₂ Cycloalkylring;
oder
(b) eins von Y und Z ist Wasserstoff und das andere ist gewählt aus der Gruppe bestehend aus
(1) Wasserstoff,
(2) Hydroxy,
(3) geschütztem Hydroxy,
und
(4) NR⁷R⁸, worin R⁷ und R⁸ unabhängig gewählt sind aus Wasserstoff und C₁-C₆ Alkyl, oder R⁷ und R⁸ werden zusammengenommen mit dem Stickstoffatom, an welches sie gebunden sind, um einen 3- bis 7-gliedrigen Ring zu bilden, der, wenn der Ring ein 5- bis 7-gliedriger Ring ist, wahlweise eine Heterofunktion enthalten kann, gewählt aus der Gruppe bestehend aus -O-, -NH-, -N(C₁-C₆-Alkyl-)-, -N(Aryl)-, -N(Aryl-C₁-C₆-alkyl)-, -N(substituiertem-Aryl-C₁-C₆-alkyl-)-, -N(Heteroaryl)-, -N(Heteroaryl-C₁-C₆-alkyl-)-, -N(substituiertem-Heteroaryl-C₁-C₆-alkyl-)- und -S- oder -S(O)ₙ-, worin n 1 oder 2 ist;
R^{a} ist Wasserstoff oder Hydroxy;
R^{b} ist Wasserstoff oder eine Hydroxyschutzgruppe;
L ist Methylen oder Carbonyl, vorausgesetzt, daß, wenn L Methylen ist, T -O- ist;
T ist gewählt aus der Gruppe bestehend aus -O-, -NH-, und -N(W-R^{d})-, worin W abwesend ist oder gewählt ist aus der Gruppe bestehend aus -O-, -NH-CO-, -N=CH- und -NH-, und R^{d} ist gewählt aus der Gruppe bestehend aus
(1) Wasserstoff,
(2) C₁-C₆ Alkyl, wahlweise substituiert mit einem oder mehreren Substituenten gewählt aus der Gruppe bestehend aus
(a) Aryl,
(b) substituiertem Aryl,
(c) Heteroaryl,'
(d) substituiertem Heteroaryl,
(e) Hydroxy,
(f) C₁-C₆ Alkoxy,
(g) NR⁷R⁸, worin R⁷ und R⁸ wie vorher definiert sind,
und
(h) -CH₂-M-R⁹,
worin M gewählt ist aus der Gruppe bestehend aus
(i) -C(O)-NH-,
(ii) -NH-C(O)-,
(iii) -NH-,
(iv) -N=,
(v) -N(CH₃)-,
(vi) -NH-C(O)-O-,
(vii) -NH-C(O)-NH-,
(viii) -O-C(O)-NH-,
(ix) -O-C(O)-O-,
(x) -O-,
(xi) -S(O)ₙ-, worin n 0, 1 oder 2 ist,
(xii) -C(O)-O-,
(xiii) -O-C(O)-,
und
(xiv) -C(O)-;
und
worin R⁹ gewählt ist aus der Gruppe bestehend aus:
(i) C₁-C₆ Alkyl, wahlweise substituiert mit einem Substituenten gewählt aus der Gruppe bestehend aus
(aa) Aryl,
(bb) substituiertem Aryl,
(cc) Heteroaryl, und
(dd) substituiertem Heteraryl;
(ii) Aryl,
(iii) substituiertem Aryl,
(iv) Heteroaryl,
(v) substituiertem Heteroaryl,
und
(vi) Heterocycloalkyl;
(3) C₃-C₇ Cycloalkyl,
(4) Aryl,
(5) substituiertem Aryl,
(6) Heteroaryl,
und
(7) substituiertem Heteroaryl;
X' ist C₃ Alkinyl;
Y' ist Thienyl;
Z' ist Pyridyl;
und A, B, D und E sind unabhängig gewählt aus der Gruppe bestehend aus:
(a) Wasserstoff;
(b) C₁-C₆ Alkyl, wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Heterocycloalkyl;
(vi) Hydroxy;
(vii) C₁-C₆ Alkoxy;
(viii) Halogen bestehend aus Br, Cl, F oder I;
und
(ix) NR⁷R⁸, worin R⁷ und R⁸ wie vorher definiert sind;
(c) C₃-C₇ Cycloalkyl;
(d) Aryl;
(e) substituiertem Aryl;
(f) Heteroaryl;
(g) substituiertem Heteroaryl;
(h) Heterocycloalkyl; und
(i) einer Gruppe gewählt aus Option (b) oben, weiter substituiert mit -M-R⁹, worin M und R⁹
wie vorher definiert sind, unter der Voraussetzung, daß mindestens zwei von A, B, D und E Wasserstoff sind;
oder irgendein Paar von Substituenten, bestehend aus AB, AD, AE, BD, BE oder DE, wird zusammengenommen mit dem Atom oder den Atomen, an welche sie gebunden sind, um einen 3- bis 7-gliedrigen Ring zu bilden, wahlweise eine Heterofunktion enthaltend gewählt aus der Gruppe bestehend aus -O-, -NH-, -N(C₁-C₆ Alkyl-)-, -N(Aryl-C₁-C₆ alkyl-)-, -N(substituiertes Aryl-C₁-C₆ Alkyl-)-, -N(Heteroaryl-C₁-C₆ Alkyl-)-, -N(substituiertes Heteroaryl-C₁-C₆ Alkyl-)-, -S- oder -S(O)ₙ-, worin n 1 oder 2 ist, -C(O)-NH-, -C(O)-NR¹²-, worin R¹² gewählt ist aus der Gruppe bestehend aus Wasserstoff, C₁-C₃ Alkyl, C₁-C₃ Alkyl substituiert mit Aryl, substituiertem Aryl, Heteroaryl oder substituiertem Heteroaryl, -NH-C(O)-, und -NR¹²-C(O)-;
worin das Verfahren folgendes umfaßt:
Koppeln einer Verbindung gewählt aus der Gruppe bestehend aus einer Verbindung von Formel I^{a} 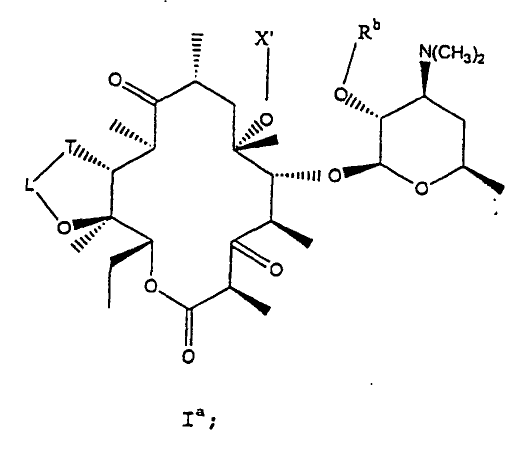 einer Verbindung von Formel II^{a} 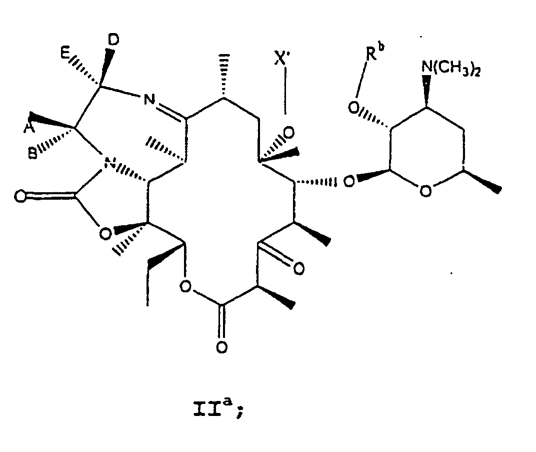 und einer Verbindung von Formel III^{a} 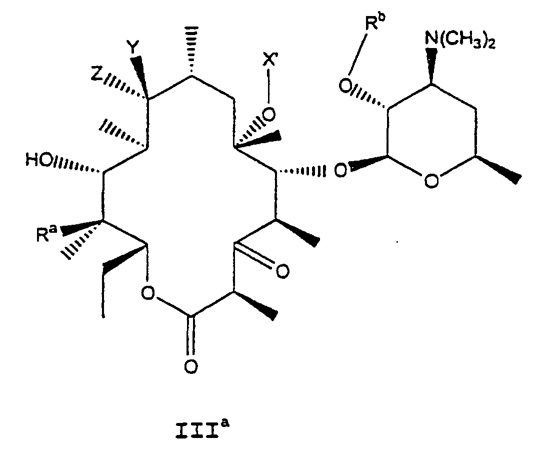 worin
R^{b} eine Hydroxyschutzgruppe ist, und
Y, Z, X, R¹, R², R³, R⁴, R⁵, R⁶, R⁷, R⁸, n, R^{a}, L, T, R^{d}, W, M, R⁹, X', Y', Z', A, B, D, E, R¹² sind wie vorher definiert;
mit einer Verbindung gewählt aus der Gruppe bestehend aus X¹-Y'-Z' und X¹-Y'-X², worin X¹ und X² unabhängig gewählt sind aus der Gruppe bestehend aus Bromid, Jodid, Sulfonat, Trialkylstannan, Boronsäure und Borat, und Y' und Z' sind wie vorher definiert; in der Anwesenheit eines Palladiumkatalysators,
unter der Voraussetzung, daß, wenn X¹-Y'-X² verwendet wird, eine nachfolgende Kopplungsreaktion mit X³-Z' durchgeführt wird, worin X³ gewählt ist aus der Gruppe bestehend aus Bromid, Jodid, Sulfonat, Trialkylstannan, Boronsäure und Borat, und Z' wie vorher definiert ist;
(b) wahlweise Deprotektieren;
und
(c) wahlweise Isolieren der gewünschten Verbindung.

## Revendications

1. Composé choisi dans le groupe constitué par un composé de formule I 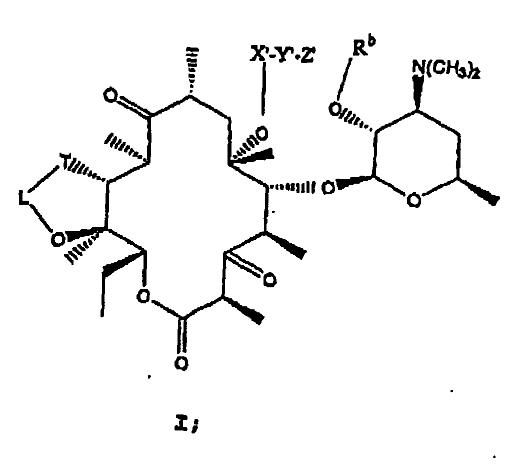 un composé de formule II 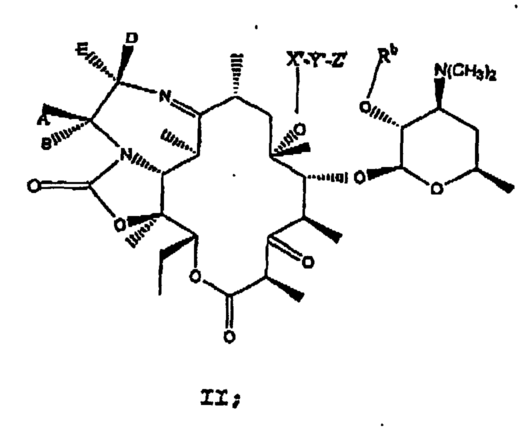 et un composé de formule III 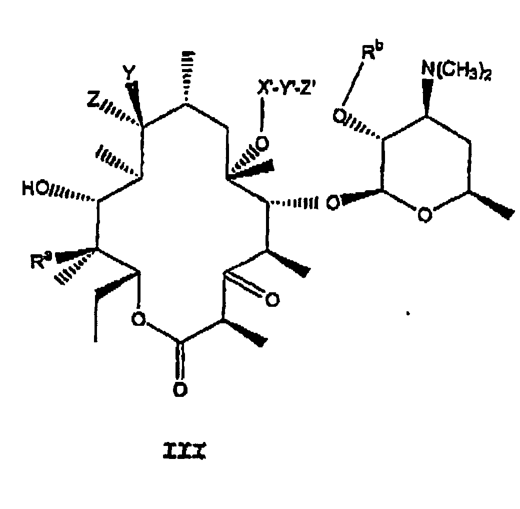 ou un sel, un solvant, un ester ou un promédicament pharmaceutiquement acceptable de ceux-ci, dans lequel :
(a) Y et Z pris ensemble définissent un groupe X, et X est choisi dans le groupe constitué par
(1) =O,
(2) =N-OH,
(3) =N-O-R¹, où R¹ est choisi parmi le groupe constitué par
(a) un groupe alkyle en C₁-C₁₂ non substitué,
(b) un groupe alkyle en C₁-C₁₂ substitué par un groupe aryle,
(c) un groupe alkyle en C₁-C₁₂ substitué par un groupe aryle substitué,
(d) un groupe alkyle en C₁-C₁₂ substitué par un groupe hétéroaryle,
(e) un groupe alkyle en C₁-C₁₂ substitué par un groupe hétéroaryle substitué,
(f) un groupe cycloalkyle en C₃-C₁₂, et
(g) -Si-(R²)(R³)(R⁴) où R², R³ et R⁴ sont chacun indépendamment choisis parmi un groupe alkyle en C₁-C₁₂ et un groupe aryle ;
et
(4) =N-O-C(R⁵)(R⁶)-O-R¹, où R¹ est tel que défini précédemment et R⁵ et R⁶ sont chacun indépendamment choisis dans le groupe constitué par
(a) l'hydrogène,
(b) un groupe alkyle en C₁-C₁₂ non substitué,
(c) un groupe alkyle en C₁-C₁₂ substitué par un groupe aryle,
(d) un groupe alkyle en C₁-C₁₂ substitué par un groupe aryle substitué,
(e) un groupe alkyle en C₁-C₁₂ substitué par un groupe hétéroaryle,
et
(f) un groupe alkyle en C₁-C₁₂ substitué par un groupe hétéroaryle substitué,
ou R⁵ et R⁶ pris ensemble avec l'atome auquel ils sont reliés forment un noyau cycloalkyle en C₃-C₁₂ ;
ou
(b) l'un de Y et Z est de l'hydrogène et l'autre est choisi dans le groupe constitué par
(1) l'hydrogène,
(2) un groupe hydroxy,
(3) un groupe hydroxy protégé,
et
(4) NR⁷R⁸, où R⁷ et R⁸ sont indépendamment choisis parmi l'hydrogène et un groupe alkyle en C₁-C₆, ou R⁷ et R⁸ sont pris avec l'atome d'azote auquel ils sont reliés afin de former un noyau à 3 à 7 éléments qui, lorsque le noyau est un noyau à 5 à 7 éléments, peut facultativement contenir une fonction hétéro choisie dans le groupe constitué par -O-, -NH-, -N(alkyle en C₁-C₆)-, -N(aryle)-, -N(arylalkyle en C₁-C₆)-, -N((aryl substitué)alkyle en C₁-C₆)-, -N(hétéroaryle)-, -N(hétéroarylalkyle en C₁-C₆)-, -N((hétéroaryl substitué)alkyle en C₁-C₆-)-, et -S- ou -S(O)ₙ, où n est 1 ou 2 ;
R^{a} est de l'hydrogène ou un groupe hydroxy ;
R^{b} est de l'hydrogène ou un groupe hydroxy-protecteur ;
L est du méthylène ou un groupe carbonyle, à condition que, lorsque L est du méthylène, T soit -O- ;
T est choisi dans le groupe constitué par -O-, -NH-, et -N(W-R^{d})-, où W est absent ou est choisi dans le groupe constitué par -O-, -NH-CO-, -N=CH- et -NH-, et R^{d} est choisi dans le groupe constitué par
(1) l'hydrogène,
(2) un groupe alkyle en C₁-C₆ facultativement substitué par un ou plusieurs substituants choisis dans le groupe constitué par
(a) un groupe aryle,
(b) un groupe aryle substitué,
(c) un groupe hétéroaryle,
(d) un groupe hétéroaryle substitué,
(e) un groupe hydroxy,
(f) un groupe alcoxy en C₁-C₆,
(g) NR⁷R⁸, où R⁷ et R⁸ sont tels que définis précédemment,
et
(h) -CH₂-M-R⁹,
où M est choisi dans le groupe constitué par
(i) -C(O)-NH-,
(ii) -NH-C(O)-,
(iii) -NH-,
(iv) -N=,
(v) -N(CH₃)-,
(vi) -NH-C(O)-O-
(vii) -NH-C(O)-NH-
(viii) -O-C(O)-NH-
(ix) -O-C(O)-O-
(x) -O-,
(xi) -S(O)ₙ-, où n est 0, 1 ou 2,
(xii) -C(O)-O-,
(xiii) -O-C(O)-,
et
(xiv) -C(O)- ;
et
où R9 est choisi dans le groupe constitué par :
(i) un groupe alkyle en C₁-C₆, facultativement substitué par un substituant choisi dans le groupe constitué par
(aa) un groupe aryle,
(bb) un groupe aryle substitué,
(cc) un groupe hétéroaryle, et
(dd) un groupe hétéroaryle substitué ;
(ii) un groupe aryle ;
(iii) un groupe aryle substitué ;
(iv) un groupe hétéroaryle ;
(v) un groupe hétéroaryle substitué,
et
(vi) un groupe hétérocycloalkyle,
(3) un groupe cycloalkyle en C₃-C₇,
(4) un groupe aryle,
(5) un groupe aryle substitué,
(6) un groupe hétéroaryle,
et
(7) un groupe hétéroaryle substitué ;
X' est un groupe alcynyle en C₃ ,
Y' est un groupe thiényle ;
Z' est un groupe pyridyle ;
et A, B, D et E sont indépendamment choisis dans le groupe constitué par :
(a) l'hydrogène,
(b) un groupe alkyle en C₁-C₆, facultativement substitué par un ou plusieurs substituants choisis dans le groupe constitué par :
(i) un groupe aryle ;
(ii) un groupe aryle substitué ;
(iii) un groupe hétéroaryle ;
(iv) un groupe hétéroaryle substitué ;
(v) un groupe hétérocycloalkyle ;
(vi) un groupe hydroxy ;
(vii) un groupe alcoxy en C₁-C₆ ;
(viii) un halogène constitué par Br, Cl, F ou I ; et
(ix) NR⁷R⁸, où R⁷ et R⁸ sont tels que définis précédemment ;
(c) un groupe cycloalkyle en C₃-C₇ ;
(d) un groupe aryle ;
(e) un groupe aryle substitué ;
(f) un groupe hétéroaryle ;
(g) un groupe hétéroaryle substitué ;
(h) un groupe hétérocycloalkyle ; et
(i) un groupe choisi dans l'option (b) ci-dessus, substitué en outre par -M-R⁹, où M et R⁹ sont tels que définis précédemment, à condition qu'au moins deux de A, B, D et E soient de l'hydrogène ;
ou toute paire de substituants, constituée par AB, AD, AE, BD, BE ou DE, est prise avec l'atome ou les atomes auxquels ceux-ci sont reliés afin de former un noyau à 3 à 7 éléments contenant facultativement une fonction hétéro choisie dans le groupe constitué par -O-, -NH-, -N(alkyle en C₁-C₆)-, -N(arylalkyle en C₁-C₆)-, -N(arylalkyle en C₁-C₆)-, -N((aryl substitué)alkyle en C₁-C₆)-, -N (hétéroarylalkyle en C₁-C₆)-, -N((hétéroaryl substitué)alkyle en C₁-C₆)-, -S- ou -S(O)ₙ, où n est 1 ou 2, -C(O)-NH, -C(O)-NR¹²-, où R¹² est choisi dans le groupe constitué par l'hydrogène, un groupe alkyle en C₁-C₃, un groupe alkyle en C₁-C₃ substitué par un groupe aryle, un groupe aryle substitué, un groupe hétéroaryle, ou un groupe hétéroaryle substitué, -NH-C(O)-, et NR¹²-C(O)-.

2. Composé selon la revendication 1, dans lequel L est un groupe carbonyle.

3. Composé selon la revendication 1, dans lequel T est -NH-.

4. Composé selon la revendication 1, dans lequel R^{b} est un groupe hydroxy-protecteur.

5. Composé selon la revendication 1, dans lequel R^{b} est de l'hydrogène.

6. Composé selon la revendication 1, dans lequel -X', Y' et Z' sont combinés afin de former un groupe R, et R est choisi dans le groupe constitué par
-(CH₂)-C=C-(5-(2-pyridyl)-2-thiényle),
-(CH₂)-C=C-(5-(3-pyridyl)-2-thiényle), et
-(CH₂)-C=C-(5-(4-pyridyl)-2-thiényle).

7. Composé selon la revendication 1 de formule I, dans lequel R^{b} est H, L est -C(O)-, T est -NH-, R est -(CH₂)-C=C-(5-(2-pyridyl)-2-thiényle).

8. Composition pharmaceutique comprenant une quantité pharmaceutiquement efficace d'un composé selon la revendication 1 en combinaison avec un véhicule pharmaceutiquement acceptable.

9. Composition pharmaceutique comprenant une quantité pharmaceutiquement efficace d'un composé selon la revendication 7 en combinaison avec un véhicule pharmaceutiquement acceptable.

10. Utilisation d'un composé selon la revendication 1 pour la préparation d'un médicament destiné à traiter une infection bactérienne chez un mammifère ayant besoin d'un tel traitement.

11. Utilisation d'un composé selon la revendication 7 pour la préparation d'un médicament destiné à traiter une infection bactérienne chez un mammifère ayant besoin d'un tel traitement.

12. Composé selon la revendication 1 pour utilisation comme agent thérapeutique.

13. Procédé de préparation d'un composé choisi dans le groupe constitué par un composé de formule I 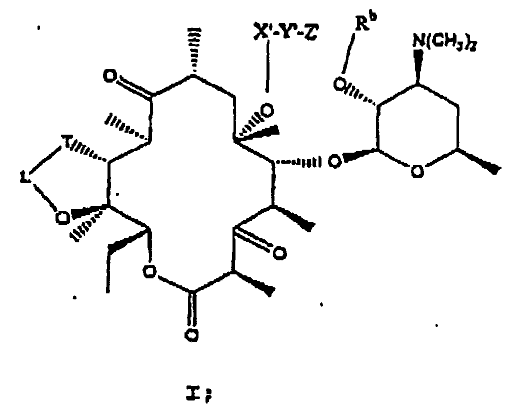 un composé de formule II 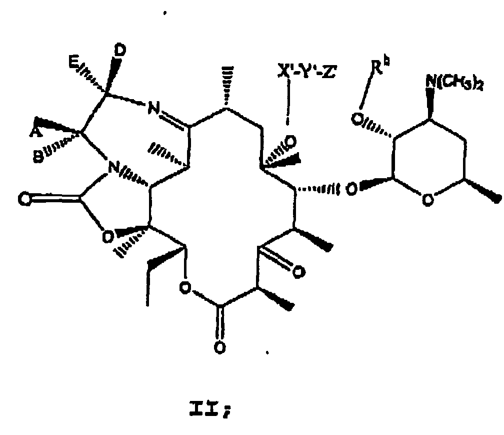 et un composé de formule III 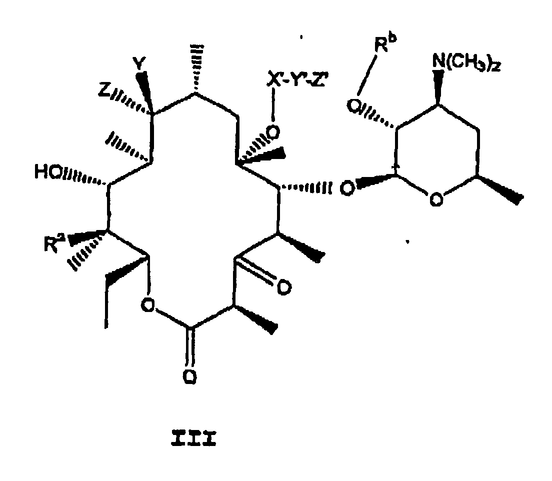 ou un sel, un solvant, un ester ou un promédicament pharmaceutiquement acceptable de ceux-ci, dans lequel :
(a) Y et Z pris ensemble définissent un groupe X, et X est choisi dans le groupe constitué par
(1) =O,
(2) =N-OH,
(3) =N-O-R¹, où R¹ est choisi dans le groupe constitué par
(a) un groupe alkyle en C₁-C₁₂ non substitué,
(b) un groupe alkyle en C₁-C₁₂ substitué par un groupe aryle,
(c) un groupe alkyle en C₁-C₁₂ substitué par un groupe aryle substitué,
(d) un groupe alkyle en C₁-C₁₂ substitué par un groupe hétéroaryle,
(e) un groupe alkyle en C₁-C₁₂ substitué par un groupe hétéroaryle substitué,
(f) un groupe cycloalkyle en C₃-C₁₂, et
(g) -Si-(R²)(R³)(R⁴), où R², R³ et R⁴ sont chacun indépendamment choisis parmi un groupe alkyle en C₁-C₁₂ et un groupe aryle ;
et
(4) =N-O-C(R⁵)(R⁶)-O-R¹, où R¹ est tel que défini précédemment et R5 et R6 sont chacun indépendamment choisis dans le groupe constitué par
(a) l'hydrogène,
(b) un groupe alkyle en C₁-C₁₂ non substitué,
(c) un groupe alkyle en C₁-C₁₂ substitué par un groupe aryle,
(d) un groupe alkyle en C₁-C₁₂ substitué par un groupe aryle substitué,
(e) un groupe alkyle en C₁-C₁₂ substitué par un groupe hétéroaryle,
et
(f) un groupe alkyle en C₁-C₁₂ substitué par un groupe hétéroaryle substitué,
ou R⁵ et R⁶ pris ensemble avec l'atome auquel ils sont reliés forment un noyau cycloalkyle en C₃-C₁₂ ;
ou
(b) l'un de Y et Z est de l'hydrogène et l'autre est choisi dans le groupe constitué par
(1) l'hydrogène,
(2) un groupe hydroxy,
(3) un groupe hydroxy protégé,
et
(4) NR⁷R⁸, où R⁷ et R⁸ sont indépendamment choisis parmi l'hydrogène et un groupe alkyle en C₁-C₆, ou R⁷ et R⁸ sont pris avec l'atome d'azote auquel ils sont reliés afin de former un noyau à 3 à 7 éléments qui, lorsque le noyau est un noyau à 5 à 7 éléments, peut facultativement contenir une fonction hétéro choisie dans le groupe constitué par -O-, -NH-, -N(alkyle en C₁-C₆), -N(aryle), -N(arylalkyle en C₁-C₆)-, -N((aryl substitué)alkyle en C₁-C₆)-, -N((hétéroaryl substitué) alkyle en C₁-C₆)-, et -S- ou -S(O)ₙ-, où n est 1 ou 2 ;
R^{a} est l'hydrogène ou un groupe hydroxy ;
R^{b} est l'hydrogène ou un groupe hydroxy-protecteur ;
L est du méthylène ou un groupe carbonyle, à condition que, lorsque L est du méthylène, T soit -O- ;
T est choisi dans le groupe constitué par -O-, -NH- et -N(W-R^{d})-, où W est absent ou est choisi dans le groupe constitué par -O-, -NH-CO-, -N=CH- et -NH-, et R^{d} est choisi dans le groupe constitué par
(1) l'hydrogène,
(2) un groupe alkyle en C₁-C₆ facultativement substitué par un ou plusieurs substituants choisis dans le groupe constitué par
(a) un groupe aryle,
(b) un groupe aryle substitué,
(c) un groupe hétéroaryle,
(d) un groupe hétéroaryle substitué,
(e) un groupe hydroxy,
(f) un groupe alcoxy en C₁-C₆,
(g) NR⁷R⁸, où R⁷ et R⁸ sont tels que définis précédemment,
et
(h) -CH₂-M-R⁹,
où M est choisi dans le groupe constitué par
(i) -C(O)-NH-,
(ii) -NH-C(O)-,
(iii) -NH-,
(iv) -N=,
(v) -N(CH₃)-,
(vi) -NH-C(O)-O-
(vii) -NH-C(O)-NH-
(viii) -O-C(O)-NH-
(ix) -O-C(O)-O-
(x) -O-,
(xi) -S(O)ₙ-, où n est 0, 1 ou 2,
(xii) -C(O)-O-,
(xiii) -O-C(O)-,
et
(xiv) -C(O)- ;
et
où R⁹ est choisi dans le groupe constitué par :
(i) un groupe alkyle en C₁-C₆, facultativement substitué par un substituant choisi dans le groupe constitué par
(aa) un groupe aryle,
(bb) un groupe aryle substitué,
(cc) un groupe hétéroaryle, et
(dd) un groupe hétéroaryle substitué ;
(ii) un groupe aryle
(iii) un groupe aryle substitué
(iv) un groupe hétéroaryle,
(v) un groupe hétéroaryle substitué
et
(vi) un groupe hétérocycloalkyle;
(3) un groupe cycloalkyle en C₃-C₇,
(4) un groupe aryle,
(5) un groupe aryle substitué,
(6) un groupe hétéroaryle,
et
(7) un groupe hétéroaryle substitué ;
X' est un groupe alcynyle en C₃ ;
Y' est un groupe thiényle ;
Z' est un groupe pyridyle ;
et A, B, D et E sont indépendamment choisis dans le groupe constitué par :
(a) l'hydrogène,
(b) un groupe alkyle en C₁-C₆, facultativement substitué par un ou plusieurs substituants choisis dans le groupe constitué par :
(i) un groupe aryle ;
(ii) un groupe aryle substitué ;
(iii) un groupe hétéroaryle ;
(iv) un groupe hétéroaryle substitué ;
(v) un groupe hétérocycloalkyle ;
(vi) un groupe hydroxy ;
(vii) un groupe alcoxy en C₁-C₆ ;
(viii) un halogène constitué par Br, Cl, F ou I ; et
(ix) NR⁷R⁸, où R⁷ et R⁸ sont tels que définis précédemment ;
(c) un groupe cycloalkyle en C₃-C₇ ;
(d) un groupe aryle ;
(e) un groupe aryle substitué ;
(f) un groupe hétéroaryle ;
(g) un groupe hétéroaryle substitué ;
(h) un groupe hétérocycloalkyle ; et
(i) un groupe choisi parmi l'option (b) ci-dessus, substitué en outre par -M-R⁹, où M et R⁹ sont tels que définis précédemment, à condition qu'au moins deux de A, B, D et E soient de l'hydrogène ;
ou toute paire de substituants, constituée par AB, AD, AE, BD, BE ou DE, est prise avec l'atome ou les atomes auxquels ceux-ci sont reliés afin de former un noyau à 3 à 7 éléments contenant facultativement une fonction hétéro choisie dans le groupe constitué par -O-, -NH-, -N(alkyle en C₁-C₆)-, -N(arylalkyle en C₁-C₆)-, -N ((aryl substitué)alkyle en C₁-C₆)-, -N(hétéroarylalkyle en C₁-C₆)-, -N((hétéroaryl substitué)alkyle en C₁-C₆)-, -S- ou -S(O)ₙ-, où n est 1 ou 2, -C(O)-NH, -C(O)-NR¹²-, où R¹² est choisi dans le groupe constitué par l'hydrogène, un groupe alkyle en C₁-C₃, un groupe alkyle en C₁-C₃ substitué par un groupe aryle, un groupe aryle substitué, un groupe hétéroaryle, ou un groupe hétéroaryle substitué, -NH-C(O)-, et NR¹²-C(O)- ;
où ledit procédé comprend :
a) le couplage d'un composé choisi dans le groupe constitué par
un composé de formule I^{a} 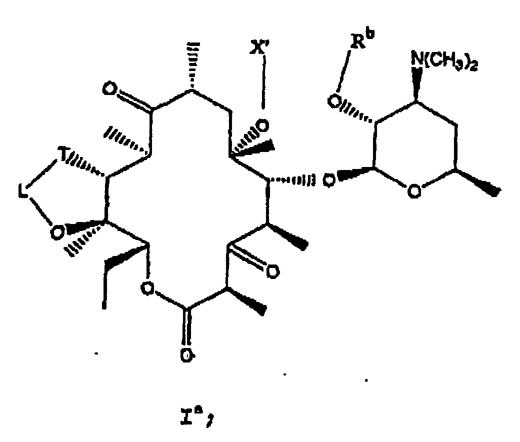 un composé de formule II^{a} 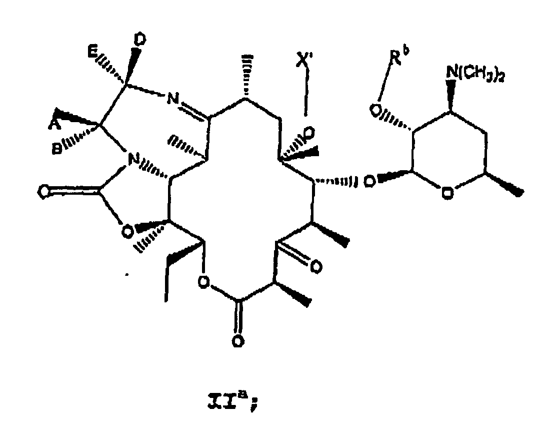 et un composé de formule III^{a} 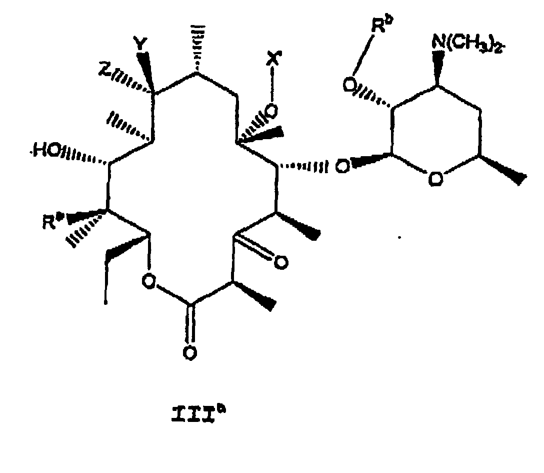 où
R^{b} est un groupe hydroxy-protecteur, et Y, Z, X, R¹, R², R³, R⁴, R⁵, R⁶, R⁷, R⁸, n, R^{a}, L, T, R^{d}, W, M, R⁹, X', Y', Z', A, B, D, E, R¹² sont tels que définis précédemment ;
avec un composé choisi dans le groupe constitué par X¹-Y'-Z' et X¹-Y'-X², où X¹ et X² sont indépendamment choisis dans le groupe constitué par le bromure, l'iode, le sulfonate, le trialkylstannane, l'acide boronique et le borate, et Y' et Z' sont tels que définis précédemment,
en présence d'un catalyseur de palladium,
à condition que, lorsque X¹-Y'-X² est utilisé, une réaction de couplage subséquente soit effectuée avec X³-Z', où X³ est choisi dans le groupe constitué par le bromure, l'iode, le sulfonate, le trialkylstannane, l'acide boronique et le borate, et Z' est tel que défini précédemment ;
(b) une déprotection faculative ;
et
(c) l'isolement facultatif du composé souhaité.
